# EUROPEAN PATENT SPECIFICATION

(11) **EP 2 271 935 B1**
(45) Date of publication and mention of the grant of the patent: **03.10.2012**
(21) Application number: 09729792.3
(22) Date of filing: 09.04.2009
(51) Int. Cl.: G01N 33/574, G01N 33/50

(54) **METHOD AND KIT FOR THE EX VIVO EVALUATION OF THE RESPONSE OF A TUMOR TO CONDITIONS TO BE TESTED**
VERFAHREN UND KIT ZUR EX-VIVO-BEURTEILUNG DER REAKTION EINES TUMORS AUF ZU TESTENDE BEDINGUNGEN
PROCÉDÉ ET KIT POUR L ÉVALUATION EX VIVO DE LA RÉPONSE D UNE TUMEUR À DES CONDITIONS À ÉVALUER

(30) Priority: 11.04.2008 US 44082 P
(43) Date of publication of application: 12.01.2011
(73) Proprietor: Universität Leipzig, 04109 Leipzig (DE); Dietz, Andreas, 04416 Markkleeberg (DE); Tschöp, Katrin, 04317 Leipzig (DE); Wichmann, Gunnar, 04299 Leipzig (DE); Granzow, Christof, 69121 Heidelberg (DE)
(72) Inventor: DIETZ, Andreas, 04416 Markkleeberg (DE); TSCHÖP, Katrin, 06849 Dessau (DE); WICHMANN, Gunnar, 04299 Leipzig (DE); GRANZOW, Christof, 69121 Heidelberg (DE)
(74) Representative: Kailuweit & Uhlemann Patentanwälte
(86) International application number: PCT/EP2009/054297
(87) International publication number: WO 2009/124997

(56) References cited:
- DOLLNER RALPH ET AL: "Ex vivo responsiveness of head and neck squamous cell carcinoma to vinorelbine" ANTICANCER RESEARCH, HELENIC ANTICANCER INSTITUTE, ATHENS, GREECE, [Online] vol. 26, no. 3B, 1 May 2006 (2006-05-01), pages 2361-2365, XP008106967 ISSN: 0250-7005
- BERTHOIS Y ET AL: "PHENOL RED IN TISSUE CULTURE MEDIA IS A WEAK ESTROGEN IMPLICATIONS CONCERNING THE STUDY OF ESTROGEN-RESPONSIVE CELLS IN CULTURE" PROCEEDINGS OF THE NATIONAL ACADEMY OF SCIENCES OF THE UNITED STATES OF AMERICA, vol. 83, no. 8, 1986, pages 2496-2500, XP002531959 ISSN: 0027-8424
- BARAK VIVIAN ET AL: "Clinical utility of cytokeratins as tumor markers" CLINICAL BIOCHEMISTRY, ELSEVIER INC, US, CA, vol. 37, no. 7, 1 July 2004 (2004-07-01), pages 529-540, XP002451710 ISSN: 0009-9120
- LUKITS J ET AL: "Molecular identification, expression and prognostic role of estrogen- and progesterone receptors in head and neck cancer", INTERNATIONAL JOURNAL OF ONCOLOGY, vol. 30, no. 1, January 2007 (2007-01), pages 155-160, ISSN: 1019-6439

## Description

The invention concerns a method for the *ex vivo* evaluation of the response of a tumor to conditions to be tested, in particular a tumor treatment regime, in an isolated tumor sample. The diagnostic method is suitable for the use in medicine and in pharmacy, in particular for the pretherapeutical evaluation of the responsiveness of a tumor to a tumor treatment regime and the preclinical evaluation of a tumor treatment regime.

Conventional colony-forming assays used on tumor probes do not distinguish between cell types and have a low reproducibility due to artifacts.

US 2001/0051353 describes a method for using multicellular particulates to analyze malignant or hyperproliferative tissue. In this conventional colony-forming assay probes or solid tumors are disintegrated mechanically and taken into culture. Of this prime culture probes are taken, subcultured and separated for further analysis to assay cytotoxic effects of chemotherapeutics or irradiation. Non-malignant cells might be taken as control culture.

Other methods perform a digest of the tumor tissue with the enzyme trypsin to yield a cell suspension. This cell suspension is than assayed for cytotoxic or other cell biological effects. This commonly used trypsin digest poses problems, as the trypsin digests important cell surface markers (*e.g*. cell-specific surface proteins), leads to a singularization of the cells, disrupts the information flow between tumor cells and stroma cells, might be toxic to some cells or types of cells and reduces the colony forming ability of the tumor cells. Consequently, the *in vitro* experiment is far away from correctly reflecting the situation *in vivo.*

One problem of colony forming assays that are performed with isolated tumor probes is the heterogeneity of the tumor probe. A tumor or carcinoma probe normally consists of different cells types, mainly stroma cells (endothelial cells and fibroblasts) and epithelial cells, all of them being able to form colonies in the colony forming assay [R. Dollner et al. Anticancer Res. 24, 325-331 (2004), R. Dollner et al. Anticancer Res. 26, 2361-2365 (2006)]. Of particular medical interest are the epithelial cells, as they form the malignant cells in carcinoma. However, the endothelial cells as target cells of angiogenesis inhibitors are also of therapeutic interest. The desired action of cytostatics leads to a suppression of colony forming ability in all cell types. The necessary dose of cytostatic varies strongly dependent on the cell type and the cytostatic used.

As already stated, the colonies present in a treated sample of a solid tumor can origin from one, two or three or even more cell types. It is possible to observe samples, in which most or all the colonies origin from stroma cells or which contains several major cell types. Thus, problems are posed by measuring the enzymatic activity or other parameters that correlate with the sum in cell number or any other tests that result in a measurement of sum response of all cell types present. The information value of such tests is low, as the results can not be attributed to a cell type and in particular the targeted malignant cell type of interest. To be useful for the clinic, a test that differentiates and correctly assigns the cell type to the colonies formed is essential. This assignment of the cell type in probes that are conventionally stained according to Giemsa is very difficult, time consuming and needs a special training. Furthermore, in the *in vitro* colony forming assay structures are formed, which are not present in the freshly isolated tumor tissue. In particular the histological appearance of the cell colonies formed is difficult to interpret, as the morphology of colonies originating form epithelial, fibroblast and endothelial cells is manifold and also other cell types might be present.

In the studies performed in the state of the art important errors are performed during sample preparation, which lead to artifacts and a poor reproducibility of the results, even if the staining by Giemsa and the histological evaluation was done correctly. The inventors have found out that this poor reproducibility is due to treatment under standard laboratory conditions in clean benches and utilization of standard cell cultivation conditions.

Consequently, the determined IC₅₀ values for cisplatin vary from publication to publication in factors between 30 and 100, for vincristin the factors of variation is even higher than 30,000. For other cytostatics the variation of IC₅₀ values is in between these extremes. IC₅₀ values are an important criterion for the description of the chemo response as they stand for the concentration of the chemotherapeutic, which reduces the proliferation of the examined cells to 50 % compared to the untreated control.

Often already the double of the tolerable doses of a cytostatics leads to exitus. Consequently, due to their constitutive low reproducibility and high variability, the above cited conventional assays are of low value for the necessities of medicine and useless for the determination of a suitable dose of a cytostatic in a chemotherapy setting.

Although tumor therapies, like irradiation and the administration of cytostatics and combined methods are already used for decades, the success rate for most carcinomas is still as low as 20 percent. A treatment with a dosage, that is chosen too low, or a treatment with the wrong chemotherapeutic is often not only lacking a positive effect, but rather having a negative effect as it leads to an enhanced chemoresistance of the tumor. Hence, the success rates of a second subsequent treatment are generally much lower as the success rate in the first treatment. The therapeutic schemes in the state of the art are more or less empirically defined, which is problematic as every tumor and its reaction to chemotherapeutics and irradiation differs and there is often only one chance to choose and effectively apply a therapy *in vivo.*

Consequently, there is still a need for methods, that can predict the outcome of a response of a tumor to conditions to be tested, in particular a tumor treatment regime, *e.g*. the response of a tumor to a chemotherapy scheme, a targeted therapy or irradiation or a combination thereof.

Objective of the invention is thus to provide a method for the *ex vivo* evaluation of a tumor treatment regime in an isolated tumor sample, which avoids artifacts, having a higher reproducibility, allowing standardization and being suitable for the pretherapeutical evaluation of the responsiveness of a tumor to a tumor treatment regime and the preclinical evaluation of a tumor treatment regime.

This objective is solved by a method for the *ex-vivo* evaluation of the response of a tumor (preferably of one individual) to conditions to be tested, in particular a tumor treatment regime or the evaluation of the response of a tumor to a substance to be tested, in an isolated tumor sample, comprising the following steps:
a.) Placing the freshly isolated tumor sample in a container, which contains antibiotics and preferably antimycotics, keeping the sample at a temperature above 10°C, preferably above 15°C,
b.) Breaking up, preferably mechanically disintegrating, the tumor sample, preferably into pieces with a size from 0.1 mm³ to 10 mm³,
c.) Placing the pieces of the tumor sample into cell culture medium that contains collagenase, within at most 12 hours, preferably 5 hours, upon isolation, incubating for at least 10 hours, preferably at least 15 hours, washing and resuspending the cells and tissue pieces,
d.) Plating the cells and tissue pieces into wells that are coated with extra cellular matrix protein(s), incubating under the conditions to be tested, in particular the tumor treatment regime or in presence of a substance (or substances) to be tested,
e.) Determining the number of tumor cells and/or the number of tumor cell colonies that are preferably from epithelial origin by performing a cytokeratin staining.

The extra cellular matrix protein or proteins are preferably added either as a mixture of extra cellular matrix proteins (e. g. a crude mixture produced by a cell line) or as purified proteins, preferably chosen form collagen, fibronectin, laminin and elastin.

The term tumor treatment as used in the description of the invention comprises single tumor treatment regimes, or a combination or a series of tumor treatment regimes (e. g. radiotherapy followed by chemotherapy or a combination of different chemotherapeutics). The method according to the invention is in particular useful to evaluate a combination or a series of tumor treatment regimes based on a tumor probe isolated from the patient before applying this combined treatment regime to the same patient.

The cell culture medium used in the steps from c.) to e.) contains less than 100 nmol/l flavin and does not contain phenol red.

The steps c.) to e.) are performed in the absence of light with a wave length below 520 nm, preferably below 530 nm, most preferably 550 nm.

**Step a.):** After isolation by biopsy or resection of the tumor the freshly isolated tumor sample is placed in a container (*e.g*. a tube), which contains antibiotics, *e.g*. penicillin, amikacin, and streptomycin, to prevent bacterial contamination and preferably antimycotics, preferably nystatin and/or other antimycotics like fluconazol. The antibiotics and antimycotics are preferably dissolved in cell culture medium (*e.g*. based on riboflavin-free RPMI 1640 without phenol red), which is described more in detail underneath. Advantageously, the addition of antibiotics and antimycotics to all media used and right before the beginning of all steps of the procedure outperforms the state of the art. The inventors have found that the addition of antimycotics does not influence the colony formation of the tumor cells. Particularly preferred is a combination of penicillin, streptomycin, amikacin, nystatin and/or fluconazol.

Upon isolation the sample is kept at a temperature above 10 °C, preferably above 15 °C and preferably below 30 °C, most preferably around or slightly above room temperature, and transported to laboratory preferably within 2 hours, most preferably within one hour.

In **step b.)** the tumor sample is broken up, preferably by mechanical disintegration into pieces of a size of about 0.1 mm³ to 10 mm³ but even slightly higher volumes, preferably up to 30 mm³, are acceptable if the pieces are small enough to ensure maintenance with nutrients and gas exchange is guaranteed. The breaking up is preferably done by mincing the sample using sterile scalpels or scissors in a scissor-like or cutter-like way (*e.g*. by chopping using two scalpels). Before or during this step most of the connecting and vascular tissue is taken away. The weight of the remaining tumor mass is determined. To avoid microbial contamination, this is preferably done using a sterile container that was weight right before weighting the tumor sample. The net weight of the sample is calculated as the difference of brut (container plus sample) and tar (container without sample).

A tumor sample of at least 50 mg, preferably of at least 100 mg, is preferred. Advantageously, the inventors have found a sample of about 100 mg to be fully representative for the whole tumor and correctly reflecting its chemoresponse, so that micro heterogeneity of the tumor mass does not pose a problem.

In **step c.)** the pieces of the tumor mass are now placed into cell culture medium (preferably pre-warmed) that contains collagenase (EC 3.4.24), preferably collagenase type IV, most preferably from *Clostridium histolyticum* (EC 3.4.24.3, type IV). This is done within at least 5 hours upon isolation of the tumor, preferably within 2 hours. The collagenase digest is performed by incubating for at least 10 hours, preferably at least 15 hours. After incubation the cells and tissue pieces are washed and re-suspended. Advantageously and opposite to the trypsin digest performed in the state of the art, the collagenase digest leaves the cells and part of the tissue matrix intact and is not toxic to the cells. The collagenase concentration ranges preferably within 100 and 200 Units/ml, most preferably within 110 to 150 Units/ml. The cell density is adjusted according to the tumor mass (determined in step b.), preferably adjusted to be around 1 to 10 mg/ml, most preferably 3 to 7 mg/ml.

In **step d.)** the resuspended cells and tissue pieces are plated into wells of a tissue culturing plate (*e.g*. a 96-well plate), preferably with a volume of 100 µl to 300 µl per well. The surface of the well has been coated with extracellular matrix or extracellular matrix proteins. The coating with extracellular matrix is preferably achieved by plating tumor cells (primary cells or cell lines, from human or animal origin, *e.g.* a mouse fibroblast cell line), which are then killed (*e.g.* by radiation) and removed (*e.g*. by scraping). Alternatively the plates are coated with a single protein or a combination of extracellular matrix components isolated from nature or produced recombinant, preferably fibronectin and/or collagen preferably dissolved in phosphate buffered saline.

Directly after plating or after a maximum of 2 hours initial incubation under cell culture conditions, the cells are incubated under the conditions to be tested, preferably under conditions of the tumor treatment regime or in presence of the substance (or substances) to be tested.

The incubation under conditions to be tested is preferably started immediately following the enzymatic disintegration by collagenase or even during the collagenase digest. If a sequential protocol of a combined therapeutic treatment is intended, this has to be integrated into the digesting step. If a delayed start of the test procedure is desired to allow a useful integration into laboratory routine, a maximum shift in starting the collagenase digest of about 12 hours is possible with only minor effects on colony formation if the immediately prepared and mechanically disintegrated tumor sample is transferred into glass ware containing an appropriate amount of the culture medium described.

The term tumor treatment regime according to the invention refers to every non surgical method to remove or to prevent the expansion of a tumor. The term tumor treatment regime in particular includes chemotherapy (exposure to one or several chemotherapeutics, including the treatment with substances from biological origin), immunotherapy and therapy with physical means, as radiation therapy, or a combination of those methods (*e.g*. combined radio-chemotherapy or radio-chemotherapy combined with the treatment with substances from biological origin or small molecules). Exposure to several chemotherapeutics means the simultaneous exposure to a combination of chemotherapeutics or the subsequent exposure to at least two chemotherapeutics alone or a combination of chemotherapeutics. The term tumor treating regime further comprises regimes with cytostatic, anti-angiogenetic or targeted therapies. The term treatment with substances from biological origin, comprises the treatment with substances as proteins like growth factors or growth factor receptors or their agonists or antagonists, antibodies or recombinant proteins, nucleic acids or derivates thereof, in particular small interfering RNA (siRNA) or vectors, like viruses or parts of viruses used for transfection. The term chemotherapy comprises not only the treatment with conventional anticancer drugs, like cytostatic drugs, but also the treatment with small molecules acting as tyrosine, serine or threonine kinase inhibitors or other substances interfering with intracellular signaling.

The incubation time, interval and concentration with chemotherapeutics is chosen dependent on the regime to be tested. Preferably the incubation is carried out over a time span from 24 h to 192 h, most preferably 72 h to 120 h.

The chemotherapeutic is preferably administered in dilution series and if necessary in a suitable diluent.

Beside testing chemotherapeutic treating regimes the method according to the invention is suitable to evaluate other non-surgical tumor treatment regimes, as radiotherapy with ionizing rays, antibodies, targeted therapies, combinations or other methods or substances inhibiting tumor growth.

As most of these methods influence the growth of tumor epithelia, the proliferation or survival of epithelial cells is taken as a read out in the method according to the invention. Also the presence of marker proteins on the surface of or even present within these cells or even a reporter gene assay in a cell line is alternatively chosen as appropriate readout. The same applies to other malignant cells or cells producing malignant cells like stem cells (SCs) including *e.g*. cancer stem cells (CSCs) or cells which are forming or are participating in forming a malignant environment.

Hormesis is a dose response phenomenon known for environmental factors that would seem to produce positive responses and have also been termed "eustress". Hormesis is characterized by a high dose inhibition, but low dose stimulation, producing an either J-shaped or an inverted U-shaped dose response. Hormetic effects of a chemotherapeutical treatment are followed by toxic effects at higher concentrations. To completely cover a dose-response curve, the analysis of a sufficient number of doses or dose combinations is needed arguing for the execution of the test in microtiter plate wells which are manually, semi-automatically or automatically handled simultaneously.

Generally preferably, at least three different doses of the treatment regime (plus control) are examined. Most preferably, especially to detect hormetic effects, eight different doses of the treatment regime (plus control) are examined.

Alternatively, the evaluation of the response of a tumor to other substances or other conditions to be tested is performed. These substances may include but are not limited to noxes of biological or chemical origin or environmental factors. Other conditions to be tested may include hypo- and/or hyperthermic as well as hypoxic or hyperoxic treatment sequences alone or combined with other treatments. If a radiation treatment is tested alone or combined with chemotherapy, the cell culturing is preferably performed in a way to reduce every possible deviation from the optimal cell culturing conditions mentioned, especially regarding temperature and pH in the culture. This is reached by reducing every break in incubation and, if transportation is necessary, to control temperature and CO₂-content during transport, *e.g*. by use of styrofoam boxes containing pre-warmed thick metal plates or other container-implemented devices with high effective heat capacity harboring the test plate during transportation and applying pre-warmed CO₂-containin air from prefilled bags via tubes into the closed boxes. The foil bags applied can be made of Mylar and aluminum, Mylar or other inert material.

After incubation under the conditions to be tested (under conditions of the tumor treatment regime or in presence of the substance(s) to be tested) the cells are prepared for the detection step e.). If necessary the probes are treated with a suitable fixation step (*e.g*. with methanol or ethanol, acetone, (para-) formaldehyde or other fixants) before staining with antibodies, fluorescence label or detection by immunohistochemistry (*e.g*. peroxidase reaction).

In **step e.)** the number of tumor cells or their response to the treatment regimen are determined. This is preferably done by detecting the number of cells and/or the number of colonies and/or the sum response of cells that are from epithelial origin by performing a cytokeratin staining. Additionally the response of a target molecule of a tumor treatment might be measured or the presence of a biomarker, *e.g*. a CSC marker or a protein or RNA that is involved in or correlates with a certain cell function.

The sum response only of epithelial cells is preferably measured using a cell-based enzyme-linked immunosorbent assay (ELISA) or radio-immunoassay (RIA) or immunofluorescent staining.

The term response of a target comprises the down or up regulation of a molecular target in response to a targeted therapy (*e.g*. a target specific antibody or siRNA, target specific inhibitors or substances exerting stimulating effects on tumor suppressive molecules). The measurement of the response of a target is preferably performed on the mRNA- or protein level, *e.g*. by using antibodies or a gene probe for *in situ* hybridization. The targets include - to name only a few - components of signal transduction pathways like serine, threonine or tyrosine kinases, components of the ras pathway, the NF-κB pathway or transcription factors like jak, STATs (*e.g*. STAT3) or NF-AT or components part of cell cycle regulation or control like ki-67, cdks and others, as well as components involved in apoptosis or inhibition of apoptosis like caspases, p53, fas etc., or bcl-2, bcl-x, bad, components of Notch- or Wnt-signaling pathway or components of the Hedgehog-signaling pathway like patch or smoothened, and others.

For the selective identification of epithelial cells (to distinguish them from stroma cells) cytokeratins, which are fibrous intermediate-filament protein polymers of the cytoskeleton of keratinocytes and epithelial cells, are detected by specific antibodies or antibody-cocktails. There are 20 so far known cytokeratins. Cytokeratins 1 to 8 are classified according to their molecular weight and isoelectric pH as neutral-basic or type II and cytokeratins 9 to 20 as acidic or type I. Cytokeratins are always expressed in specific pairs of one unit of type I and one unit of type II. Optimal results are achieved using pan-cytokeratin antibodies, but limited success is achievable using individual anti-cytokeratin antibodies which offer the possibility to fine-differentiate the detected cell. In general, the cytokeratin staining allows the identification of epithelial cells or epithelial cell colonies, and to clearly distinguish them from stroma cells and stroma cell colonies.

Preferably anti-cytokeratin antibodies specific for individual cytokeratin are used, in order to achieve a fine-differentiation of particular kinds of epithelial cells. In particular antibodies specific for cytokeratin 8 and 18 are preferably used to detect adenocarcinoma-derived epithelial cells or colonies or for distinguishing hyperplasia and dysplasia. Further antibodies specific for cytokeratin 4 and 13 are preferably used to detect in particular malignant forms of oral mucosal and esophageal epithelial cells.

The label is preferably a fluorescence marker (for the detection in a fluorescence microscope), a tag like biotin or an enzyme (like peroxidase or phosphatase), that can catalyze a color-producing reaction (which can be used depended on the substrate used for the detection in a normal microscope or in an ELISA reader).

Preferably, the detection of cells from epithelial origin is done using a microscope by counting the number of colonies formed. Alternatively, the anti-cytokeratin staining can be measured photometrically with a dedicated measuring device, *e.g*. in an ELISA reader, spectrophotometer or luminometer, to measuring the total number of tumor cells or a signal proportional to the number of tumor cells.

The identification cells from epithelial origin is done by staining with an anti-cytokeratin antibody, which is either directly labeled or which is preferably detected by a labeled secondary antibody, *e.g*. an anti-mouse-Cy2, anti-mouse-Cy3, anti-mouse-horseradish peroxidase or anti-mouse-alkaline phosphatase.

The fluorescent-labeled cells can be screened using a suitable fluorescence microscope. The fluorescence dye should be chosen dependent on the equipment. Alternatively, the antibody can be detected without a fluorescence microscope using conventional immunohistochemistry (*e.g*. using the peroxidase reaction).

For the evaluation of the response towards a treatment preferably the number of colonies formed per well are counted. Alternatively the intensity of the color/fluorescence or the area occupied by the colonies is determined and compared to the values of untreated controls carried along the assay. In this way a dose-response relation is obtained, which allows to calculate suitable parameters, as IC₅₀-values for the chemotherapeutic or a combination therapy.

The colonies formed by spreading out of the tissue clumps of the tumor sample correspond pretty much to metastases, which would probably occur in the *in vivo* situation. The colonies can be of low number with bigger size or high number and smaller size (per colony). Thus the information achieved by counting the colonies and measuring the total number of tumor cells normally differs.

Advantageously, both methods (counting and ELISA/RIA) can be combined. Following fluorescence staining and counting of epithelial cells the second evaluation can be done by using an enzyme-labeled antibody also detecting the cytokeratin-bound primary antibody and the subsequent use of chromogenic substrate and extinction measurement.

The cell culture medium used in the steps c.) to e.) is a modified standard cell culture medium (like RPMI 1640) supplemented with 5 to 20%, preferably 10 % (v/v) serum, *e.g*. fetal calf serum or human serum or - if obtainable - autologous serum), that in general contains in sum less than 100 nmol/l flavin, preferably less than 50 nmol/l, most preferably 10 to 20 nmol/l as final concentration in culture. Thus the flavin content mentioned is the total content, including the riboflavin present in the serum added. The term flavin includes riboflavin and other substances, wherein a flavin moiety is attached to a nucleotide (like FAD, FADH or FADH₂) or other moiety (like riboflavin-5-phosphate). Preferably the cell culture medium is also free of dyes like phenol red. Alternatively a preferably phenol-red-free serum-free medium (*e.g.* a medium which is designed for culturing stem cells) is used that has a reduced amount of flavin as described above.

Preferably the buffer concentrations are adapted to 12 to 16 mmol/l, most preferably 14 to 15 mmol/l HEPES (4-(2-hydroxyethyl)-1-piperazineethanesulfonic acid), and 12 to 15 mmol/l, most preferably 13 to 14 mmol/l NaHCO₃. The medium preferably further contains 1 to 2 mmol/l of stabilized glutamine. To prevent bacterial contamination, the medium preferably further contains antibiotics, as penicillin, streptomycin and amikacin, and preferably also antimycotics, as nystatin and/or fluconazol or other antimycotics. Particularly preferred is a combination of penicillin, streptomycin, amikacin and nystatin.

The cell culture conditions in step c.) to e.) are preferably adapted to a CO₂-content below 4% (v/v), most preferably from 3.4 to 3.6% CO₂ instead of the normally used 5%. The atmosphere is humidified and preferably adapted to a temperature from 36.4 °C to 36.6°C. By lowering the CO₂-content in the atmosphere the pH-value in the cell culture medium is defined and adapted to the modified cell culture medium in order to avoid artifacts.

The steps c.) to e.) and preferably also step b.) are performed in the presence of yellow light only, meaning the absence of light with a wavelength below 520, preferably below 530 nm, preferably only light with a wave length above 550 nm being present. This is preferably achieved by using sodium discharge lamps or LED (*e.g*. with 589 nm emission only within a small band pass, *e.g*. 589 ± 5 nm). The containers used in step a.) are preferably of darkened glass or any other material that prevents blue light and UV-light to enter or, if not, the container with the tumor is inserted into and transported in a container or bag that is impermeable to light. Preferably the lamp used in the microscope used for inspections of the tumor test plates is equipped with a yellow filter.

The term tumor according to the invention refers to a malignant neoplasm in which a group of cells displays the traits of uncontrolled growth (growth and division beyond the normal limits), invasion (intrusion on and destruction of adjacent tissues), and sometimes metastasis (spread to other locations in the body via lymph or blood). In the context of this application tumors are in particular carcinoma, thus a cancer of epithelial origin. Preferably the tumor sample of epithelial origin is taken from a carcinoma, preferably chosen from squamous cell carcinomas, papillomas, adenomas or adenocarcinomas, anaplastic carcinomas (*e.g*. of head and neck cancer), adnexal and skin appendage cancer, cystic, mucinous, and serous carcinoma, ductal, lobular and medullary cancer, acinar cell cancer, complex epithelial cancer, gonadal cancer, paragangliomas and glomus tumors, nevi and melanomas and other types of carcinomas. Examples include larynx, pharynx, oro- and hypopharynx tumors, breast cancer, lung cancer, cervix cancer, hepatocellular cancer, skin cancer like basal cell carcinoma and Merkel carcinoma and others.

Within the method according to the invention the elimination of photochemical artifacts has been made possible by employing flavin-protecting conditions that include an adapted cell culture medium and handling the sample and all media including sera in the dark or under light with long wave length and avoiding short wavelength light in particular below 520 nm. This results in an enhancement of the reproducibility of the assays to comply with clinical needs.

The method of the invention is in particular characterized by the following features:

### Obtaining and preparation of the tumor sample:

Tumor samples are obtained by biopsy or during tumor resection and transferred immediately without fixation into a prepared test tube and are transported to the laboratory within a short period of time. There arrived, the samples are first weighed and then processed mechanically. During the processing of tumor samples temperatures below room temperatures are to be avoided. Data regarding the tumor sample are documented thoroughly.

### Ex vivo testing/examination:

In the method according to the invention samples of isolated tumor are tested directly after mechanical disintegration and subsequent collagenase digest without cultivating the samples *in vitro* or passage. By that method, selection caused by cultivation as well as adaptation of the test samples (tumor and stroma cells) to the *in vitro* environment will be avoided. This is done to emulate the examined cellular reactions with as few disturbing influences as possible, that is, to guarantee that the *ex vivo* test results can also be applied to the *in vivo* situation.

### Non-tryptic (tissue) digestion:

By using collagenase instead of trypsin, artifacts which are otherwise inevitable are avoided. The non-tryptic tissue digestion does not destroy interactions between the cells of explanted tumor tissue. So the isolated cell and tissue structures (e.g. epithelial complexes and vascular endothelium) remain functional because they are allowed to remain in their native microenvironment together with those cells which are *in vivo* adjacent to them. Decollating and singularization of the cells, which would prevent cells from communicating with each other, is specifically avoided by the collagenase digest. After the tissue digestion cells are washed with cell culture medium and the pellet (mainly consisting out of tissue clumps or pats) is resuspended, adjusted to the desired concentration, and added into the prepared micro-well plates.

### Elimination of photochemical artifacts:

Photochemical artifacts are caused by photoactivation of flavins, especially riboflavin, in cell culture media. Flavin-mediated photoreactions are one of the main causes for low reproducibility of conventional cytotoxicity tests. Photoactivation of flavins leads to chaotropism and irreproducible results, making it impossible to determine the chemoresponse in a way that reflects the situation *in vivo* correctly. The method according to the invention prevents artifacts caused by the photoactivation of flavins by defining flavin-protecting assay conditions and a cell culture medium adjusted to these conditions. These flavin-protecting assay conditions comprise the exclusive use of nearly flavin-free cell culture media and working in the absence of light with short wavelength, *e.g*. microscopy of cell cultures using yellow filters. These measures effectively prevent the photoactivation of flavins, leading to an optimal reproducibility of the test results. In addition to the reduction of photochemical artifacts by the medium used and the conditions according to the invention (low flavin concentration and absence of light) advantageously avoid the light-dependent cytotoxicity of flavins.

### Colony formation on ECM-coated substrate and inclusion of stroma cells:

The bottom of the wells of the micro-well plates used for the assay is coated with extracellular matrix (ECM). This is preferably achieved by growing tumor cells on the bottom of these wells, which are then killed and removed. The coating of the wells with components of extracellular matrix not only promotes the adherence of tumor cells, but also sustains the presence of stroma cells during the assay. The presence of stroma cells during the assay supports the correct expression of sensitivity and resistance characteristics of tumor cells and raises their ability to form colonies *in vitro* significantly. The complete procedure is designed not just to warrant the presence of cells of the tumor stroma, *e.g*. endothelial cells and fibroblasts, during all steps of the assay, but also their proliferation and formation of colonies. Thus the stroma cells are able to enable the correct expression of sensitivity or resistance characteristics of the coexisting epithelial tumor cells. This is crucial for the validity of the test results.

### Advantages of the analysis of the colony formation assay by immunodetection:

By specifically staining the tumor epithelial cells using anti-cytokeratin-antibody analysis of ex-vivo colony forming/formation assay are highly simplified. By specifically staining the medically relevant epithelial cell colonies they are clearly distinguished from the co-present endothelial and fibroblast colonies. Epithelial cells are visualized specifically by using cytokeratins as epithelial cell marker. Thus, the reliability of medical findings is highly improved by excluding individual factors in the evaluation of test results.

In contrast to the methods known from the state of the art the test results of the method according to the invention are not biased. As the results are obtained faster, the method of the invention saves time. The distinction between the different cell types allows a fundamentally improved depiction and record of test results.

Immunodetection can be performed either by immunofluorescence staining or staining by enzymatic reactions, e.g. an antibody-conjugated peroxidase reaction. While Peroxidase-staining represents a permanent and reliable technique, use of immunofluorescence simplifies the digital analysis of ex-vivo colony formation assays and allows the quantification and even half-automatic analysis of assays.

### Quality control:

Particularly preferred is a quality control carried out by parallel submitting a cell line to the tumor treating regime (*e.g*. dilution series of the chemo therapeutic) under cell culture conditions described above. The cell line is preferably a human tumor cell line from epithelial origin, preferably selected is the human epithelial cell line KB. In each case the cell line is preferably adapted to the flavin-protecting cell culture conditions and especially the flavin-reduced and phenol red-free medium used in assaying the tumors or metastases by culturing the cell line in the medium used for several passages (preferably 3 to 10). Alternatively other commercially available cell lines might be used. To ensure the transferability of results obtained using such cell lines, preferably cell lines should be used which are of human origin like H60, CaCo2, CFPEo, CFSMEo, HeLa, SiHa, SKOV3, MCF-7, 56FHTE8o CLBPEC PC-3, Panc-1, HepG2, and UT-SCC-16A,UT-SCC-16B, UT-SCC-54A, UT-SCC-54B, UT-SCC-54C, UT-SCC-42A, UT-SCC-42B, UT-SCC-12A, UT-SCC-12B, UT-SCC-19B, UT-SCC-66 and other carcinoma-derived cell lines. If other cell types like endothelial cells are assayed, an endothelial cell line should be used as quality control. Endothelial cell lines or cell lines of endothelial origin are HLMEC, HUVEC, H-MVEC, EA.hy926, ECY304 or others. If the response of fibroblast is to be assayed, human fibroblast cell lines like HEK-293 or HS74 should be analyzed as quality control.

Preferably the determination of the proliferation rate of the cell line is determined by counting the cells. This counting is preferably done using an automatic or semi-automatic cell counter, *e.g*. a device supplied by Coulter, by Scharfe Systems or others. Alternative procedures are possible if fulfilling the requirements regarding reproducibility. Intra- and inter-comparability of alternative procedures or different methods are preferably assured before comparison of obtained data. By analysis of the dose-response curve and their descriptive parameters (especially IC₅₀), the reproducibility of the test conditions and the applied concentrations or doses as well as a correct duration over the whole *ex vivo* testing time can be ensured.

Preferably the inhibitory concentrations (IC) of cytostatic drugs leading to diminished proliferation or even cytotoxicity and cause a drop down in cell counts to 50% of cell numbers counted in control wells receiving only medium (IC₅₀) are calculated and/or are compared to the previously obtained and/or published IC₅₀ values for the cell line(s) under flavin-protecting conditions and according to the method of the invention.

The tumor treatment regime for the particular control cell line might be adapted to the response characteristics of the control cell line (*e.g*. the concentration of the chemotherapeutic or the radiation dose).

Several control cell lines as mentioned above can be subjected in parallel to the treatment.

The method according to the invention, especially when combined with the quality control method advantageously allows the use of the outcome for diagnostic purposes.

If deviations are detected from standard IC₅₀-values, a correction factor (CF) can be calculated. This CF can be used to correct the IC₅₀ values obtained for the individual tumor to define the precise IC₅₀ concentration for the given treatment. From this CF-corrected value for an IC₅₀ the adaptation of the therapeutic treatment regime is possible. The CF is chosen in an acceptable range in relation to the IC₅₀, preferably 0.1 - 10-fold the IC₅₀, most preferably 0.5 - 2.0-fold the established IC₅₀ for the treatment according to the method of the invention.

### Additional staining of endothelial cells:

As stated above the endothelial cells present in tumors are also able to form colonies during the ex-vivo-examination. Analogous to the above described staining of the epithelial cells, the endothelial cells can be detected in parallel. This is done in a preferred variant of the method according to the invention, preferably by staining with antibodies recognizing endothelial cell markers. This variant is especially attractive in respect to the pre-therapeutic evaluation of the response of the endothelial cells of a patient's tumor to angiogenesis inhibitors, which can be substances from chemical or biological origin, or anti-angiogenesis methods.

Preferred antibodies detecting endothelial cells are antibodies binding to Factor VIII (Von-Willebrandt-factor), CD31 (PECAM-1), CD51/61 (vitronectin receptor), CD102 (ICAM-2), or CD105 (endoglin). The primary antibody for detection of endothelial cells is preferably from a different species than the anti-epithelial antibody. The secondary antibody detecting the endothelial-bound antibody should preferably contain a different label, *e.g*. a different fluorescent label than the secondary antibody to detect the anti-epithelial antibody.

If the number of endothelial cells is also determined as described above, preferably also a quality control for the staining of endothelial cells is carried out by parallel submitting an endothelial cell line to the tumor treating regime (*e.g*. dilution series of the chemotherapeutic) under cell culture conditions described above. For this quality control the cell line is preferably a human tumor cell line from endothelial origin, preferably selected from HDMEC, HCAEC, HLMEC, HUVEC, H-MVEC, EA.hy926, ECY304 and obtained from ATCC, Promocell or other suppliers.

### Additional staining of fibroblasts

It is especially useful to additionally detect fibroblasts in the method according to the invention. Fibroblasts are a special important type of stroma cells since a regulatory interaction of two or more different cell types is a constitutive property of solid tumors and in particular carcinomas. Such interaction was described for fibroblasts and epithelial cells and as well for endothelial and epithelial cells during angiogenesis. Thus the knowledge about differentially affected colony formation of these major cell types which might be exerted by the treatment is very desirable. Hence the use of a variety of cell-type specific antibodies each conjugated with a different fluorescent dye with distinct optical properties for distinct staining of these cell populations is of advantage. The detection of fibroblasts is preferably performed by staining with antibodies for vimentin or CD90 (Thy-1). The antibody is preferably labeled with a fluorescent dye - for instance Cy3 - having distinct optical properties compared to Cy2 that might be used for labeling the antibody applied for staining of cytokeratins in epithelial cells.

### Additional staining of cancer stem cells (CSCs)

According to the cancer stem cell hypothesis the CSCs are those cells of a tumor that have stem cell (SC) features such as self-renewal, drug resistance, high migration capacity, and aberrant differentiation which constitute the heterogeneous population of tumor. CSCs are known in diverse types of leukemia and also solid tumors like breast, brain, prostate, testis, ovary, stomach, lung, colon, skin, liver, and pancreas and cell lines origin from these types of cancer. They appear to be derived from a small pool of long-lived tissue-specific SCs which are defined by their ability to self-renew and to produce the well differentiated and functional cells within an organ. Differentiated cells are generally short-lived. Therefore, SCs are necessarily involved in and essential for tissue development, replacement, and repair. Because of their longevity, SCs are susceptible to accumulate genetic damage and are thereby representing the growth root for cancer recurrence following treatment. It was reported that some of the CSCs can survive chemotherapy and support recurrence and re-growth of the tumor mass. This type of tumor cells surviving chemotherapy are the so-called drug-induced or drug-selected stem cells (DSC). CSCs (including DSC) thus are a great challenge in tumor treatment. While standard chemotherapy and radiotherapy only kill dividing cells, CSCs normally survive. CSCs appear to be immortalized. They are able to renew themselves, and might develop into different cell types. CSCs origin either from normal (non-cancerous) tissue SCs or might be derived from pluripotent, multipotent or oligopotent SCs, which might have migrated to and adapted to the tumor's environment. Alternatively CSCs or CSC-like cells might be origin from genomic instable cancer cells by mutations, in particular DNA-fusion processes, and might be present following treatment in the remaining tissue as DSC. In all cases the CSC are characterized by dividing asymmetrically into one cell retaining SC characteristics and one cell being a short-lived "normal" tumor cell. Often a subpopulation of cancer cells in a given tumor or tumor cell line is described as "side population" (SP cells). These SP cells are especially rich in CSC. At presence, CSC including DSC can only efficiently be removed by surgery. If CSCs including DSC are not removed by surgery or the patient's immune system, they can be the origin of a secondary tumor and recurrent disease.

Therefore it remains a great challenge to develop tumor treatment methods that kill (most of the time non-dividing) CSC and in particular - as second line treatment - the DSCs. In order to address this problem, in the method of the invention the presence of CSCs is preferably detected in parallel to the detection of the cells of epithelial origin (and if applicable of endothelial or mesenchymal origin and/or fibroblast).

CSC, including DSC, are preferably detected by negative staining with DNA dyes, thus by their non-responsiveness to DNA dyes, preferably Hoechst 33342 or other DNA binding, in particular DNA intercalating dyes. While the majority of tumor cells of a given tumor or a cell line are stained by DNA dyes, like Hoechst 33342, these agent is efficiently excluded by CSC (including DSC) demonstrating their unique capacity to get rid of potentially dangerous compounds due to their increased expression of adenosine triphosphate-binding cassette proteins like ABCG2 (ATP-binding cassette, sub-family G (WHITE), member 2). Thus, the non-stained population of tumor cells can be determined to be CSC. This DNA staining can favorably be applied to detect CSC in various types of tumors.

Additionally or alternatively the CSC are detected by identifying stem cell markers by antibodies. The detection is preferably done by staining with antibodies recognizing human stem cell markers, preferably single markers or a combination of markers specific for hematopoietic (HSC), endothelial (ESC), mesenchymal (MSC), stromal, neural (NSC) stem cells or epithelial stem cells (ESC).

To detect HSC-like CSCs specifically preferably antibodies recognizing human CD34, CD117, Sca-1 (stem cell antigen 1) and lin (linage antigen) are used. The HSC-like CSCs are characterized by absence or low expression of CD34, expression of CD117 and Sca-1, as well as absence ofLin (CD34^{-/low}, CD117⁺, Sca-1⁺, Lin⁻).

To detect NSC-like CSCs specifically preferably antibodies recognizing human Sox-2 and Nestin are used. The co-expression of Sox2 and Nestin is defining the NSC-like CSCs.

To detect MSC-like CSCs specifically preferably antibodies recognizing human CD34, CD44, CD45 CD29 and CD90 are used. The MSC-like CSC are characterized by expression of CD44 and CD29, as well as CD90 in the absence of CD34 and CD45 (CD44⁺, CD29⁺, CD90⁺ CD34⁻, CD45⁻).

To detect ESC-like CSCs specifically preferably antibodies recognizing human CD44, EPCAM and CD117 (c-kit), are used.

Alternatively or additionally antibodies are used that detect general CSC markers, preferably antibodies directed to ABCG2 (ATP-binding cassette, sub-family G (WHITE), member 2, or CDw338) and aldehyde dehydrogenase 1 (ALDH1).

The antibodies used for detection of CSCs (as well as effects of the treatment toward them) are either a labeled antibody or an unlabeled primary antibody. If a primary labeled antibody is used it is preferably one derived from a different species than the anti-epithelial antibody and as well as the anti-endothelial antibody. If an unlabeled primary antibody is used, this one is detected by a labeled secondary antibody. The usage of antibodies of a different species is in particular necessary if a fluorescent-labeled immunoreactant that binds to the former used detecting antibody was applied. The label or labels should preferably be different to the labels used before to detect the anti-epithelial and anti-endothelial antibodies.

For the detection of CSCs several antibodies are preferably used in combination either with different labels or by using a sequential staining. In particular when a sequential staining is performed preferably a software assisted imaging analysis is used (preferably as described in US6,169,816). The detection is preferably performed by microscopy or flow cytometry or a combination thereof. This can be performed using microscopes like the Axiovert optical bench from Carl Zeiss, or other devices the like that are equipped with all necessary components like objectives, high-resolution camera, and capability for data acquisition, storage, and image processing to merge the results of the sequential analyses. Otherwise also an integrated device like the Array Scan HCS Reader (Cellomics/ ThermoFisher) or the like can be used for imaging and analysis of expression of markers in CSCs and DSCs. This approach might be based on a combination of microscopy and flow cytometry methods e.g. in a 96-well or every other possible microtiter plate format. The advantages of such approach can include a) much less cells which are needed than for any other approach as e.g. flow cytometry analysis, b) possibility of multispectral fluorescence micro-imaging that c) can be automated, and d) images can be stored, visualized and analyzed using software applications. This might also be done in an automated way.

If the number of CSC and in particular DSC is also determined as described above, preferably also a quality control for the staining of SCs is carried out by parallel submitting a cell line with stem cell properties to the tumor treating regime (e.g. dilution series of the chemotherapeutic or the compound or the biological targeting this cell) under cell culture conditions described above. For this quality control the cell line is preferably a human cell line with stem cell properties, thus a cell line with a sufficient proportion of cells with SC-like properties, preferably a sufficient frequency of CSC or SP-cells. This cell line with stem cell properties is preferably selected from human carcinoma cell lines like FaDu, A549, OVCAR3, MCF7, H460, and others.

Preferably one or more of the steps of the method according to the invention are at least partially automated. Preferably a computer is programmed to automatically or semi-automatically perform the analyses to gain information about the chemoresponse of an individual tumor. Preferably a computer is programmed to automatically perform calculations from measurement data to automatically or partially automatically analyze the chemoresponse. Such automation comprises preferably one or several of the steps of preparation of cell culture plates and especially the coating with extracellular matrix or partial procedural steps thereof as well as pipetting the reagents to be analyzed into the wells of the test plates, the harvesting of supernatants and the subsequent analysis of these supernatants or the tumor cells themselves via lysis and further analysis of expressed proteins or mRNA, automated staining procedures, and measuring using colorimetric, luminometric or scintillation measuring using any optical device analytic machine and the evaluation of results obtained during the procedure like calculation of IC₅₀ values, and other parameters describing the dose-response curve that might be used for a diagnosis of the chemoresponse and to define a treatment regime.

Another object of the invention is the use of a kit for the *ex-vivo* evaluation of a tumor treatment regime in an isolated tumor sample in the method according to the invention comprising:
a.) cell culture medium containing less than 100 nmol/l flavin, preferably being free of dyes like phenol red,
b.) an anti-cytokeratin antibody and preferably a labeled secondary antibody to detect the anti-cytokeratin antibody

Optional additional components of the kit are chosen from:
c.) an antibody, that reacts specific with human endothelial cells and preferably a labeled secondary antibody to detect the anti-endothelial cell antibody,
d.) an antibody, that reacts specific with human fibroblast cells and preferably a labeled secondary antibody to detect the anti-fibroblast antibody
e.) a DNA dye, like Hoechst 33342, and/or one or several antibodies that react specific with human stem cell markers or CSC markers.
f.) a control cell line from epithelial origin and if applicable a control cell line derived from endothelial origin and/or fibroblast cells or a fibroblast cell line.

The composition of the cell culture medium, the anti-cytokeratin antibody and the labeled secondary antibody and flavin concentration are preferably chosen as described more in detail above.

To detect endothelial cells in parallel the kit preferably contains additionally an antibody, that reacts specific with human endothelial cells and a labeled secondary antibody to detect the anti-endothelial cell antibody. These antibodies are preferably chosen as described more in detail above.

To detect CSCs the kit preferably contains as further component a DNA dye, like Hoechst 33342, and/or an antibody or a combination of antibodies that react specific with human stem cell markers (e.g. antibodies binding selectively to CD29, CD34, CD44, CD44v6, CD45, CD90, CD117, CD133, Lin, Nestin, Sca-1, Sox2, and others) and/or CSC-makers (e.g. ABCG2 and ALDH1). The antibodies recognizing CSCs are preferably chosen as described more in detail above. The antibodies are either labeled or are used as primary antibodies that are detected by labeled secondary antibodies. In the latter case the kit preferably contains additionally a secondary antibody to detect the primary anti-CSC antibody.

For quality control the kit further contains a control cell line from epithelial origin and if applicable a control cell line from endothelial origin and/or a fibroblast cell line and/or a cell line with stem cell properties as described more in detail above.

The kit may further comprise a manual describing the method according to the invention and preferably software that automates one or more of the steps of the method according to the invention or the calculation of the results at least partially. A further object of the invention is the use of the method according to the invention in medicine, in particular for the pretherapeutical evaluation of the responsiveness of a tumor to a tumor treatment regime. The invention is especially useful to determine the response of an individual tumor of an individual patient ex vivo to a tumor treatment regime, before applying the tumor treatment regime to the patient.

Another object of the invention is the use of the method according to the invention in pharmacy, in particular for the preclinical evaluation of a tumor treatment regime. The method and kit are in particular useful to examine treatment regimes, which combine classical tumor treatment regimes (cytostatics and/or irradiation) with the administration of angiogenesis inhibitors from chemical or biological origin, or anti-angiogenesis methods. The method according to the invention and the kit are further peculiarly useful to examine the interaction of cytostatics with other pharmaceuticals.

The method according to the invention and the kit advantageously allow to design and evaluate new therapeutic schemes, which are based on findings and data acquired using the method according to the invention.

The invention is further illustrated, but not thereby limited, by the examples given below.

### Example 1:

After obtaining patient's informed consent, biopsy specimens were taken from histologically confirmed primary head and neck squamous cell carcinomas during definitive surgical treatment procedures or by biopsy under general anesthesia and, after storing for less than an hour in test tubes containing antimycotics and antibiotics (nystatin, penicillin, streptomycin, and amikacin), were cleaned and weighed and were processed immediately. Thereafter the tumors which usually have a size of about 0.1 to 1 ml were mechanically disintegrated by mincing to tissue pats of about 0.1 to 10 mm³ and immersed each in 30 ml of pre-warmed riboflavin-free RPMI 1640 medium without phenol red and containing 14.5 mmol/l HEPES, 13.5 mmol/l NaHCO₃, stabilized glutamine, 10% (v/v) fetal calf serum, the antibiotics penicillin, streptomycin and amikacin, as well as the antimycotic nystatin. The tumor cells were always handled at room temperature or warmer and never exposed to light other than the light of a sodium discharge lamp with an emission wavelength of 589 nm. Immediately after mechanical disintegration 120 U/ml collagenase type IV of *Clostridium histolyticum* (EC 3.4.24.3, type IV) were added and the mixture incubated for 16 h at 36.5°C in brown glass bottles in an incubator. To ensure optimal incubation conditions, the CO₂ concentration therein was constantly kept at 3.5% and relative humidity at 95%. Following enzymatic disintegration by collagenase the digest was centrifuged for 10 min at 1200 rpm, the resulting pellets were carefully re-suspended in fresh culture medium supplemented as described but without collagenase. Thereafter, a defined amount of digested tumor (1 - 2 mg in 150 µl tumor medium) was added to microwells coated with extracellular matrix (Paesel & Lorey, Hanau, Germany) 150 µl of culture medium containing twice the concentration of docetaxel or cisplatin or no cytostatic substance at all (control). *Ex-vivo* testing was performed 72 h using the same culture conditions as during the collagenase digest.

After 72 hours of incubation, the wells were rinsed twice with phosphate buffered saline, pH 7.8, and the adherent cells and cell colonies were fixed with ice-cold 90% methanol for 10 min. Fixed specimens were exposed for 5 min to PBS/ 0.1% Tween-20^{®} (Polysorbate 20) before preincubation with 1% serum in PBS/ 0.1% Tween-20 for 45 min, followed by overnight incubation at 4°C with the primary antibody (anti-cytokeratin; SantaCruz sc-8018, C11) diluted 1:400 in PBS/ 0.1% Tween-20 supplemented with 0.2 % serum. After three rinses for 10 min each with PBS/ 0.2 % Serum/ 0.1% Tween-20, specimens were incubated for another 2 h at room temperature in the dark with the secondary antibody (anti-mouse IgG coupled with Cy2 or Cy3 fluorescence dye, Jackson ImmunoResearch, Cambs, UK) in a 1:400 dilution in PBS/ 0.2 % Serum/ 0.1 % Tween-20 followed by three rinses with PBS/ 0.2 % Serum/ 0.1 % Tween-20, and a final rinse with distilled water for 2 min. The test plate was air dried, and fluorescence signals were imaged with the Zeiss Axiovert 200M inverted fluorescence microscope (Zeiss, Jena, Germany) using Zeiss filter sets 44 and 43HE for Cy2 and Cy3, respectively.
The evaluation of the colonies is possible using every given fluorescence microscope but is eased by the use of dedicated filter sets. Colonies of epithelial origin can be clearly distinguished from stromal cells without fluorescent staining. The test is utilized by separately counting of colonies of different types (stained epithelial, unstained stromal cells), the numbers of colonies in the different treatments (*e.g*. doses of radiation or concentrations of chemotherapeutics) are recorded and kept for further analysis.

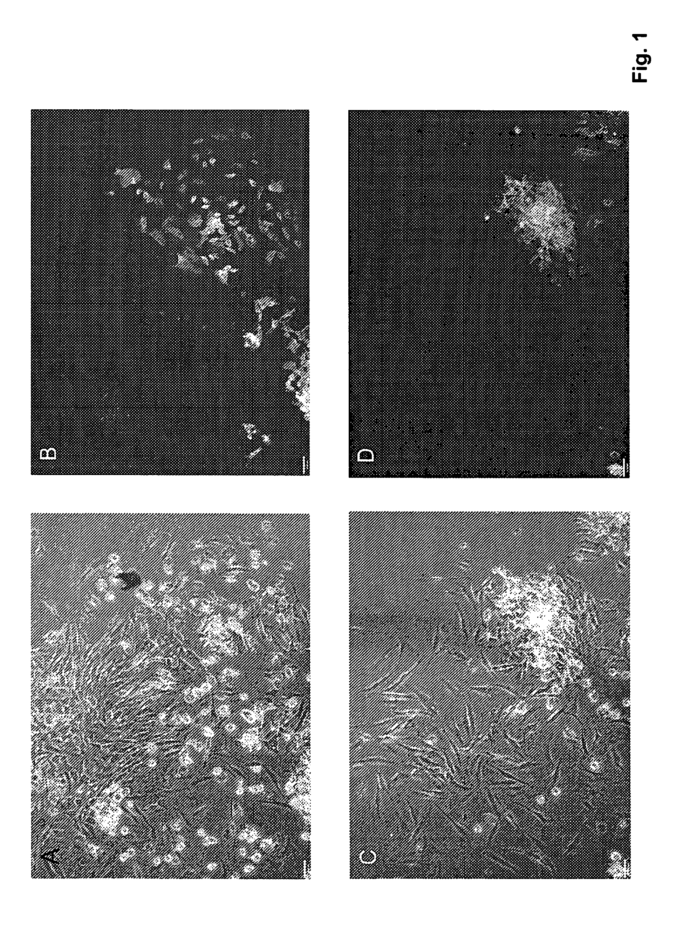

**Fig. 1** demonstrates the detection of epithelial cell colonies formed *ex vivo* according to the invention by immunofluorescence staining in the absence of cytostatic drugs
Shown is a parallel presentation of phase contrast (A and C) and Cy3 fluorescence images (B and D). Comparison of A with B and C with D indicates the selective identification of epithelial cells by immunofluorescence. No interference with coexisting stromal cells ensures improved differentiability and minimized subjectivity in analysis of the specific chemoresponse of epithelial or stromal cells *e.g*. regarding colony formation. Magnification 100-fold; bar, 54 µm.

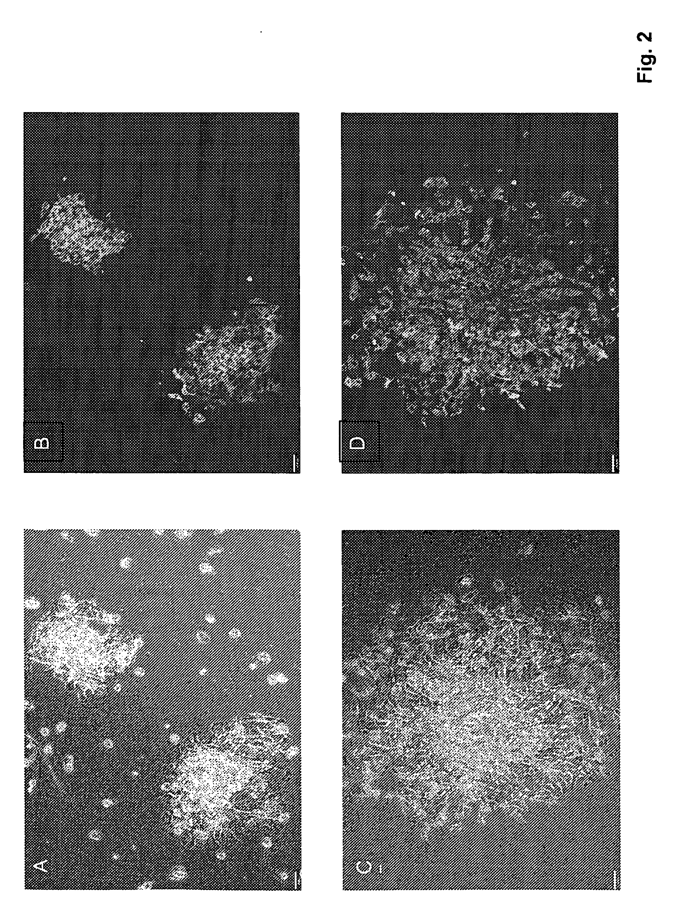

**Fig. 2** demonstrates the immunodetection of epithelial cell colonies formed *ex vivo* according to the invention by immunofluorescence staining following exposure to the cytostatic drugs cisplatin and docetaxel, respectively.
Shown is a parallel presentation of phase contrast (images A and C) and using the fluorescence filter for Cy2 fluorescence (images B and D). B: Identification of epithelial cells in the particular fluorescence image following administration of 2.25 µmol/L docetaxel. D: Detection of epithelial colonies following administration of 3.35 µmol/L cisplatin. Magnification 100-fold; bar, 54 µm.

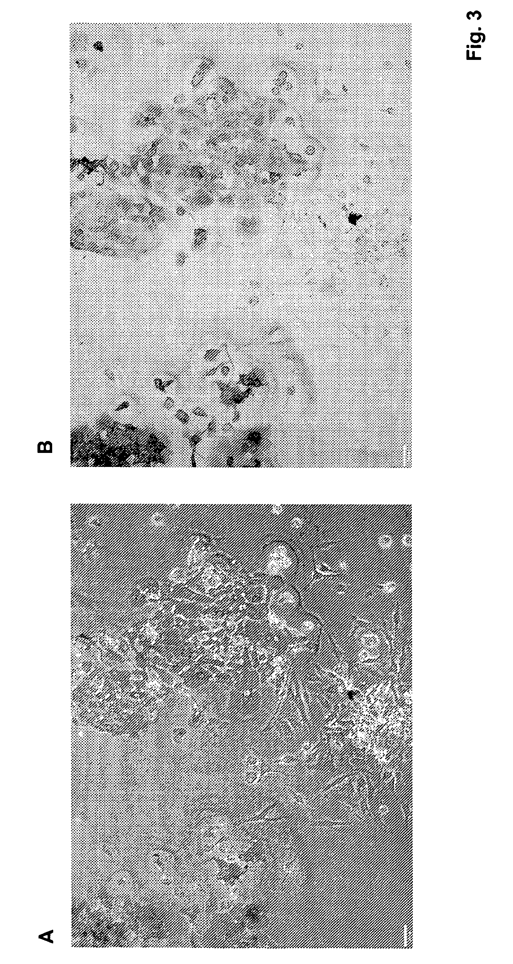

Also the use of a secondary anti-mouse-IgG-antibody conjugated with peroxidase allows immunoperoxidase staining of epithelial cell colonies. Using DAB (diaminobenzidine) for immunoperoxidase staining an alternative method to improve differentiability and minimized subjectivity in analysis of the specific chemoresponse of epithelial or stromal cells is developed. Using this alternative method results in selective brown-staining of epithelial cell colonies (Fig. 3). Fixation, primary antibody treatment and subsequent rinsing of specimens was as described above but followed by incubation for 1 h at room temperature with the biotinylated anti-mouse secondary antibody (BioGenex, diluted 1:20 in PBS/ 0.2% serum/ 0.1% Tween-20). The specimens were then rinsed twice using PBS/ 0.2% serum/ 0.1% Tween-20, and incubated for 1 h at room temperature with streptavidin-conjugated peroxidase (BioGenex, 1:20 diluted in PBS/0.2% serum/ 0.1% Tween-20). After two rinses using PBS/ 0.2% serum/ 0.1% Tween-20, epithelial colonies were identified by peroxidase oxidation of the DAB substrate, and the sufficiently stained specimens were rinsed for 2 min with distilled water. Air-dried specimens could be imaged using an arbitrarily chosen type of light-optical microscope. Epithelial cell colonies are selectively stained brown and are clearly to distinguish from stromal cells and colonies which are free of staining. The test is utilized by separately counting of colonies of different types (stained epithelial, unstained stromal cells), the numbers of colonies in the different treatments (*e.g*. doses of radiation or concentrations of chemotherapeutics) are recorded and kept for further analysis.

**Fig. 3** demonstrates the improved detectability of epithelial cell colonies formed *ex vivo* using immunodetection by immunoperoxidase staining according to the invention. Shown is a parallel presentation of phase contrast (A) and bright field (B) images after DAB staining through immunoperoxidase. Brown-staining identifies epithelial cells. Coexisting stromal cells remain unstained. Magnification 100fold; bar, 54 µm.

### Example 2a:

Freshly resected probes of head and neck squamous carcinoma of 14 untreated patients were investigated and treated if not stated otherwise as described in example 1. Three fragments were each excised from different tumor areas, weighed, and treated with collagenase as outlined in Example 1. Collagenase digests were exposed to concentrations in the range from 0.5- to 32-fold the tolerated plasma levels (TPL) of cisplatin (TLP = 6.67 µmol/l).

If the tests showed an average of ≥ 2 colonies of epithelial cells per control well, they could be used to determine cut-off concentrations (*i.e*. cisplatin concentrations required to totally suppress formation of epithelial colonies), or if ≥ 4 colonies of epithelial cells per control well, they could be used to determine IC₅₀ values (*i.e*. the half maximal inhibitory concentration of a substance), respectively (see Table 1). In some tumors (no. 2 and 7) only two samples could be evaluated. Sample wet weight averaged 146 ± 59 mg (range: 65 to 668 mg). 1.5 mg of tumor was applied to a well.

### Testing the efficiency of Cisplatin-Treatment:

Eight tumors met the criteria for IC₅₀ determination, i.e. an average of ≥ 4 colonies of epithelial cells per control well. Throughout, cut-off was reached at identical concentrations in 3 samples of 5 tumors (No. 1, 2, 5, 6 and 7). In 2 (no. 3 and 4) tumors, 2 of the samples were identical, with the 3^{rd} sample deviating only by ± 1 gradient step. Only in one tumor (No. 8) cut-off concentrations varied by more than one gradient steps.

The ranges of concentrations with log-linear relation to response quantified the microheterogeneity of the cisplatin response in a given tumor, and reached from 0.5- to 4.0-fold TPL. IC₅₀ values ranged from 0.5- to 1.91-fold TPL.

**Table 1:**

| Tumor-No. | Cut-off concentrations [n-fold TPL] | | | Range [n-fold TPL] | | IC₅₀ [n-fold TPL] | | |
|---|---|---|---|---|---|---|---|---|
| | Sample 1 | Sample 2 | Sample 3 | from | to | Sample 1 | Sample 2 | Sample 3 |
| 1 | 4 | 4 | 4 | 0.5 | 4.0 | 1.907 | 1.625 | 1.254 |
| 2 | 4 | 4 | 4 | 0.5 | 4.0 | - | 1.156 | 0.862 |
| 3 | 4 | 2 | 2 | 0.5 | 2.0 | 0.724 | 0.844 | 0.784 |
| 4 | 4 | 4 | 2 | 0.5 | 2.0 | 0.950 | 0.794 | 1.164 |
| 5 | 2 | 2 | 2 | 0.5 | 2.0 | 0.831 | 0.749 | 0.500 |
| 6 | 4 | 4 | 4 | 0.5 | 2.0 | 0.515 | 0.595 | 0.600 |
| 7 | 4 | 4 | 4 | 0.5 | 4.0 | 0.688 | - | 1.000 |
| 8 | 8 | 8 | 2 | 0.5 | 4.0 | 1.000 | 1.014 | 1.000 |

### Conclusions:

The microheterogeneity of the inhibitory effects of cisplatin was quantified *ex vivo.* Inhibitory effects observed in separately processed samples taken from different areas of individual tumors were almost identical, suggesting that the chemoresponse in a single biopsy sample is representative for the entire tumor.

### Example 2b:

Freshly resected probes of head and neck squamous carcinoma of 14 untreated patients were investigated and treated if not stated otherwise as described in example 1. Three fragments were each excised from different tumor areas, weighed, and treated with collagenase as outlined in Example 1. Collagenase digests were exposed to concentrations in the range from 0.5- to 32-fold the tolerated plasma levels (TPL) of docetaxel: (TPL = 0.55 µmol/l).

If the tests showed an average of ≥ 2 colonies of epithelial cells per control well, they could be used to determine cut-off concentrations (*i.e*. docetaxel concentrations required to totally suppress formation of epithelial colonies), or if ≥ 4 colonies of epithelial cells per control well, they could be used to determine IC₅₀ values (*i.e*. the half maximal inhibitory concentration of a substance), respectively (see Table 2). In some tumors (no. 4, 5, 8 and 9) only two samples could be evaluated.
Sample wet weight averaged 146 ± 59 mg (range: 65 to 668 mg). 1.5 mg of tumor was applied to each well.

### Testing the efficiency of docetaxel treatment:

At concentrations ≤ TPL, seven tumors (No. 2, 3, 4, 5, 6, 8 and 9) showed hormesis, i.e. treatment with docetaxel advanced/did not inhibit proliferation of tumor cells but increase the number of epithelial colonies grown. Cut-off was reached in 2 tumors only (No. 1 and 7) and, at identical or similar concentrations in the samples of a given tumor (Tab. 2). Where cut-off concentrations are marked with an X (see Tab. 2), not more than 5 colonies at most have appeared at the highest dose rate. Where cut-off concentrations are marked with an XX, more than 5 colonies have appeared at the highest dose rate.

Nine tumors met the criteria for IC₅₀ determination. Ranges of concentrations with log-linear relation to response (Tab. 2) reached from 0.5- to 32-fold TPL. IC₅₀ values determined in the individual samples of the 9 tumors are shown in Tab. 2. IC₅₀ values ranged from 0.58-fold TPL to 27.82-fold TPL. Hormesis expression varied within tumors.

**Table 2:**

| Tumor-No. | Cut-off concentrations [n-fold TPL] | | | Range [n-fold TPL] | | IC₅₀ [n-fold TPL] | | |
|---|---|---|---|---|---|---|---|---|
| | Sample 1 | Sample 2 | Sample 3 | from | to | Sample 1 | Sample 2 | Sample 3 |
| 1 | 8 | 8 | 8 | 2.0 | 8.0 | 4.565 | 4.718 | 5.040 |
| 2 | X | X | X | 0.5 | 32.0 | 1.486 | 0.579 | 1.157 |
| 3 | X | X | X | 2.0 | 32.0 | 16.404 | 10.711 | 19.173 |
| 4 | XX | X | XX | 4.0 | 32.0 | 27.819 | - | 23.393 |
| 5 | X | XX | X | 0.5 | 32.0 | 3.891 | - | 9.987 |
| 6 | X | X | X | 2.0 | 32.0 | 8.208 | 6.690 | 8.404 |
| 7 | 32 | 32 | 32 | 2.0 | 32.0 | 3.616 | 3.629 | 2.692 |
| 8 | 32 | 32 | X | 1.0 | 32.0 | 6.709 | - | 5.282 |
| 9 | X | X | X | 0.5 | 32.0 | 6.964 | 7.495 | - |

### Conclusions:

Hormetic effects of a chemotherapeutical treatment were observed so far only in a subgroup of tumors receiving docetaxel in the *ex-vivo* test according the invention. The (stimulatory) hormesis zone of docetaxel concentrations reaches from 0.5- to 4-fold TPL (tolerated plasma level, 0.55 µM). It is often but not in general followed by inhibition of colony formation at higher docetaxel concentrations. Hormesis was associated with comparably high intratumoral variability.

### Example 3:

Following an initial staining of epithelial cells using immunofluorescence, the introduction of a second antibody into the evaluation of the outcome of the *ex vivo* testing according the invention opens the possibility to answer questions regarding the effectiveness of a treatment or the underlying mechanisms that are involved in detail. Such a second antibody detecting intracellular or membrane-bound receptors or intracellular components of signal transduction pathways (which are *e.g*. involved in progression or maintenance of malignancies and therefore are targets or possible targets of chemotherapeutics or are from diagnostic value since they are able to indicate if there is responsiveness of an individual tumor towards an applied treatment) can be realised be a fluorescent labelled primary antibody or an primary antibody of another species or isotype which is detected by a fluorescent labelled secondary antibody specifically binding to antibodies of this second type. An example is given in which the effects of cisplatin on the expression of the epidermal growth factor-receptor by epithelial cells was analysed following the invention.

After obtaining patient's informed consent, biopsy specimens were taken from histologically confirmed primary head and neck squamous cell carcinomas during definitive surgical treatment procedures or by biopsy under general anesthesia and, after storing for less than an hour in test tubes containing antimycotic and antibiotics (nystatin, penicillin, streptomycin, and amikacin), were cleaned and weighed and were processed immediately. Thereafter the tumors which had a size of about 0.150, 0.650 and 0.850 ml were mechanically disintegrated by mincing to tissue pats of about 0.1 to 10 mm³ and immersed each in 30 ml of pre-warmed riboflavin-free RPMI 1640 medium without phenol red and containing 14.5 mmol/l HEPES, 13.5 mmol/l NaHCO₃, stabilized glutamine, 10% (v/v) fetal calf serum, the antibiotics penicillin, streptomycin and amikacin, as well as the antimycotic nystatin (tumor medium). The tumor cells were always handled at room temperature or warmer and never exposed to light other than the light of a sodium discharge lamp with an emission wavelength of 589 nm. Immediately after mechanical disintegration 120 U/ml collagenase type IV of *Clostridium histolyticum* (EC 3.4.24.3, type IV) were added and the mixture incubated for 16 h at 36.5°C in brown glass bottles in an incubator. To ensure optimal incubation conditions, the CO₂ concentration therein was constantly kept at 3.5% and relative humidity at 95%. Following enzymatic disintegration by collagenase the digest was centrifuged for 10 min at 1200 rpm, the resulting pellets were carefully re-suspended in fresh culture medium supplemented as described but without collagenase. Thereafter, a defined amount of digested tumor (1-2 mg in 100 µl tumor medium) was added to microwells coated with extracellular matrix (Paesel & Lorey, Hanau, Germany) and 200 µl of tumor medium containing 150% of the indicated concentration of docetaxel, cisplatin or a combination of docetaxel and cisplatin or no cytostatic substance at all (control). *Ex-vivo* testing is performed 72 h using the same culture conditions as during the collagenase digest.

After 72 hours of incubation, the wells were rinsed twice with phosphate buffered saline, pH 7.8, and the adherent cells and cell colonies were fixed with ice-cold 90% methanol for 10 min and air dried.

Fixed specimens were exposed for 5 min to PBS/ 0.1% Tween-20 before preincubation with 1% serum in PBS/ 0.1% Tween-20 for 45 min, followed by overnight incubation at 4°C with the primary antibody (anti-cytokeratin; SantaCruz sc-8018, C11) diluted 1:400 in PBS/ 0.1% Tween-20 supplemented with 0.2 % serum. After three rinses for 10 min each with PBS/ 0.2 % Serum/0.1% Tween-20, specimens were incubated for another 2 h at room temperature in the dark with the secondary antibody (anti-mouse IgG coupled with Cy2 fluorescence dye, Jackson ImmunoResearch, Cambs, UK) in a 1:400 dilution in PBS/ 0.2 % Serum/ 0.1 % Tween-20 followed by three rinses with PBS/ 0.2 % Serum/ 0.1 % Tween-20, and a final rinse with distilled water for 2 min. This was followed by overnight incubation at 4°C with the (second) primary antibody (anti-epidermal growth factor-receptor, anti-EGFR; Santa Cruz Biotechnology sc-03-G) diluted 1:400 in PBS/ 0.1% Tween-20 supplemented with 0.2 % serum. Following rinsing with PBS/ 0.2% serum/ 0.1% Tween-20, the specimens were incubated for 1 h at room temperature with the Cy3-labelled anti-goat secondary antibody (BioGenex HK337-5G, diluted 1:100 in PB S/ 0.2% serum/ 0.1% Tween-20). The specimens were then rinsed threefold using PBS/ 0.2% serum/ 0.1% Tween-20, and then air dried. The fluorescence signals were imaged with the Zeiss Axiovert 200M inverted fluorescence microscope (Zeiss, Jena, Germany) using Zeiss filter sets 44 and 43HE for Cy2 and Cy3, respectively.

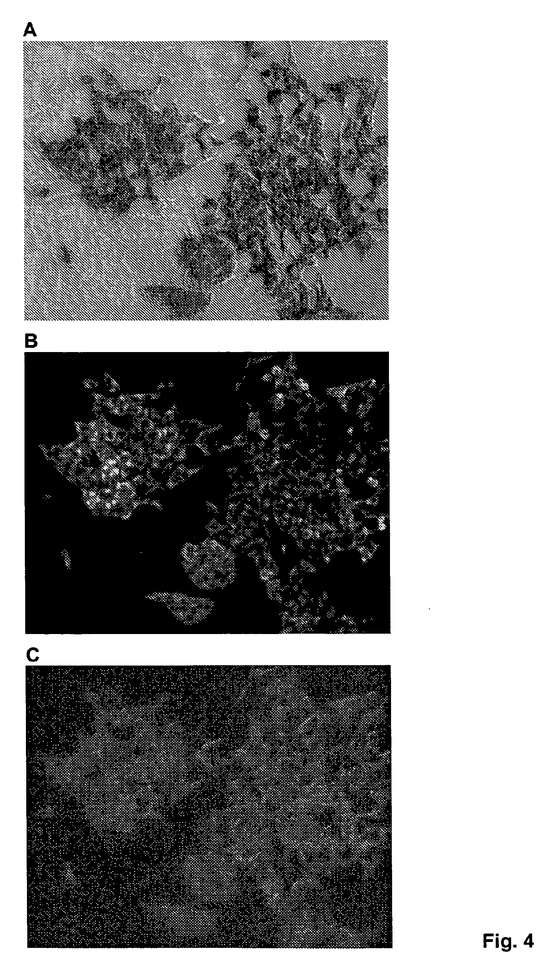

**Fig. 4** shows the simultaneous staining of cytokeratin and EGFR. The figures demonstrate the immunodetection by immunofluorescence staining of epithelial cell colonies using Cy2 (middle) and of EGFR using Cy3 labeled antibodies in colonies formed *ex vivo* following exposure to cisplatin according to the invention.
Shown is a parallel presentation of phase contrast (image A) and using the fluorescence filter for Cy2 fluorescence (image B) and Cy3 fluorescence (image C). Identification of epithelial cells (image B) also expressing EGFR (image C) following administration of 1.67 µmol/L cisplatin. 100-fold magnification.

### Example 4: Cell-based ELISA:

A cell-based ELISA to analyse the sum response of epithelial cells was performed using tumor samples of head and neck cancer. After obtaining patient's informed consent, biopsy specimens were taken from histologically confirmed primary head and neck squamous cell carcinomas during definitive surgical treatment procedures or by biopsy under general anesthesia and, after storing for less than an hour in test tubes containing the antimycotic nystatin and antibiotics (penicillin, streptomycin, and amikacin), were cleaned and weighed and were processed immediately. Thereafter the tumors which had a size of about 0.150, 0.650 and 0.850 ml were mechanically disintegrated by mincing to tissue pats of about 0.1 to 10 mm³ and immersed in 30 ml of pre-warmed riboflavin-free RPMI 1640 medium without phenol red and containing 14.5 mmol/l HEPES, 13.5 mmol/l NaHCO₃, stabilized glutamine, 10% (v/v) fetal calf serum, the antibiotics amikacin, penicillin and streptomycin, as well as the antimycotic nystatin. The tumor cells were always handled at room temperature or warmer and never exposed to light other than the light of a sodium discharge lamp with an emission wavelength of 589 nm. Immediately after mechanical disintegration 120 U/ml collagenase type IV of *Clostridium histolyticum* (EC 3.4.24.3, type IV) were added and the mixture incubated for 16 h at 36.5°C in brown glass bottles in an incubator. To ensure optimal incubation conditions, the CO₂ concentration therein was constantly kept at 3.5% and relative humidity at 95%. Following enzymatic disintegration by collagenase the digest was centrifuged for 10 min at 1200 rpm, the resulting pellets were carefully re-suspended in fresh culture medium supplemented as described but without collagenase. Thereafter, a defined amount of digested tumor (1-2 mg in 100 µl tumor medium) was added to each microwell coated with extracellular matrix (Paesel & Lorey, Hanau, Germany) and 200 µl of tumor medium containing 150% of the indicated concentration of docetaxel, cisplatin or a combination of docetaxel and cisplatin or no cytostatic substance at all (control). *Ex-vivo* testing is performed 72 h using the same culture conditions as during the collagenase digestion.

After 72 hours of incubation, the wells were rinsed twice with phosphate buffered saline, pH 7.8, and the adherent cells and cell colonies were fixed with ice-cold 90% methanol for 10 min and air dried.

Fixed specimens were exposed for 5 min to PBS/ 0.1% Tween-20 before pre-incubation with 1% serum in PBS/ 0.1% Tween-20 for 45 min, followed by overnight incubation at 4°C with the primary antibody (anti-pan-cytokeratin; Santa Cruz Biotechnology sc-8018, C11) diluted 1:400 in PBS/ 0.1% Tween-20 supplemented with 0.2 % serum. Following rinsing with PBS/ 0.2% serum/ 0.1% Tween-20, the specimens were incubated for 1 h at room temperature with the biotinylated anti-mouse secondary antibody (BioGenex HK335-5M, diluted 1:100 in PBS/ 0.2% serum/ 0.1% Tween-20). The specimens were then rinsed twice using PBS/ 0.2% serum/ 0.1% Tween-20, and incubated for 1 h at room temperature with streptavidin-conjugated peroxidase (BioGenex CJ-1005-96, 1:100 diluted in PBS/ 0.2% serum/ 0.1% Tween-20). After three rinses using PBS/ 0.2% serum/ 0.1% Tween-20, 100 µl of TMB substrate (TMB reagent set, BD Biosciences) were added and incubated in the dark at room temperature in a wet chamber. Following one hour incubation, the peroxidase-catalyzed conversion of the colourless substrate to a blue dye, the reaction was terminated by adding 100 µl of 1 mol/L H₂SO₄ inducing a change to yellow colour. The strength of enzymatic activity and the proportional extinction at a wavelength of 450 nm correlates with the number of anti-cytokeratin antibodies bound to epithelial cells and therefore can be used as measure for the impact of treatments on proliferation of epithelial cells.

Using antibodies specific for a given cell type, *e.g*. endothelial cells or fibroblasts, an analogous cell-based ELISA can be conducted to analyse cell-population or subset specific proliferation, cytostasis, or even cytotoxicity to gain information about cell-type specific effects of a given tumor treatment.

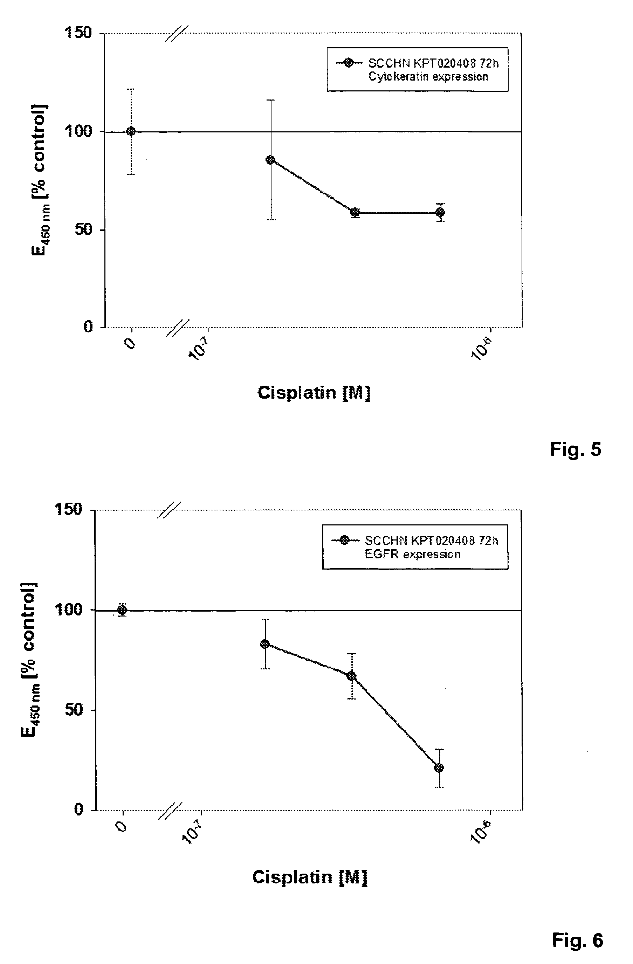

**Fig. 5** shows the effects of different doses of cisplatin on epithelial cells of a head and neck squamous cell carcinoma (HNSCC) in the *ex vivo* assay and the cell-based ELISA described in the invention. HNSCC cells were cultured 72 hours, methanol fixed and stained using the indicated pan-cytokeratin antibody (Santa Cruz Biotechnology sc-8018, C11), the biotinylated link-antibody (BioGenex HK335-5M) and the streptavidin-horseradish peroxidase conjugate (BioGenex CJ-1005-96). TMB was used as substrate and incubated for 30 min before termination by adding an equal volume (100 µl) of 1 mol/l H₂SO₄. The presented data are results of the measurement of optical density of the resulting yellow dye at 450 nm normalized to mean of control. Each measurement at the indicated concentrations of cisplatin or controls was done in six replicates for which mean and standard error of mean are shown.

### Example 5: Cell-based ELISA for the measurement of expression of membrane-bound or intracellular targets in targeted therapies

This modified version of a cell-based ELISA as given in example 3 utilizes an antibody detecting intracellular or membrane-bound receptors or intracellular components of signal transduction pathways which are involved in progression or maintenance of malignancies and therefore are targets or possible targets of chemotherapeutics or are from diagnostic value since they are able to indicate if there is responsiveness of an individual tumor towards an applied treatment. An example is given in which the effects of cisplatin on the expression of the epidermal growth factor-receptor were analysed in a cell-based ELISA following the invention.

After obtaining patient's informed consent, biopsy specimens were taken from histologically confirmed primary head and neck squamous cell carcinomas during definitive surgical treatment procedures or by biopsy under general anesthesia and, after storing for less than an hour in test tubes containing antimycotic and antibiotics (nystatin, penicillin, streptomycin and amikacin), were cleaned and weighed and were processed immediately. Thereafter the tumor which had a mass of about 150 mg was mechanically disintegrated by mincing to tissue pats of about 0.1 to 10 mm³ and immersed in 30 ml of pre-warmed riboflavin-free RPMI 1640 medium without phenol red and containing 14.5 mmol/l HEPES, 13.5 mmol/l NaHCO₃, stabilized glutamine, 10% v/v fetal calf serum, the antibiotics penicillin and streptomycin, and amikacin as well as the antimycotic nystatin. The tumor cells were always handled at room temperature or warmer and never exposed to light other than the light of a sodium discharge lamp with an emission wavelength of 589 nm. Immediately after mechanical disintegration 120 U/ml collagenase type IV of *Clostridium histolyticum* (EC 3.4.24.3, type IV) were added and the mixture incubated for 16 h at 36.5°C in brown glass bottles in an incubator. To ensure optimal incubation conditions, the CO₂ concentration therein was constantly kept at 3.5% and relative humidity at 95%. Following enzymatic disintegration by collagenase the digest was centrifuged for 10 min at 1200 rpm, the resulting pellets were carefully re-suspended in fresh culture medium supplemented as described but without collagenase. Thereafter, a defined amount of digested tumor (1-2 mg in 100 µl tumor medium) was added to microwells coated with extracellular matrix (Paesel & Lorey, Hanau, Germany) and 200 µl of tumor medium containing 150% of the indicated concentration of docetaxel, cisplatin or a combination of docetaxel and cisplatin or no cytostatic substance at all (control). *Ex-vivo* testing is performed 72 h using the same culture conditions as during the collagenase digestion.

After 72 hours of incubation, the wells were rinsed twice with phosphate buffered saline, pH 7.8, and the adherent cells and cell colonies were fixed with ice-cold 90% methanol for 10 min and air dried.

Fixed specimens were exposed for 5 min to PBS/ 0.1% Tween-20 before pre-incubation with 1% serum in PBS/ 0.1% Tween-20 for 45 min, followed by overnight incubation at 4°C with the primary antibody (anti-epidermal growth factor-receptor, anti-EGFR; Santa Cruz Biotechnology sc-03-G) diluted 1:400 in PBS/ 0.1% Tween-20 supplemented with 0.2 % serum. Following rinsing with PBS/ 0.2% serum/ 0.1% Tween-20, the specimens were incubated for 1 h at room temperature with the biotinylated anti-goat secondary antibody (BioGenex HK337-5G, diluted 1:100 in PBS/ 0.2% serum/ 0.1% Tween-20). The specimens were then rinsed twice using PBS/0.2% serum/ 0.1% Tween-20, and incubated for 1 h at room temperature with streptavidin-conjugated peroxidase (BioGenex CJ-1005-96, 1:100 diluted in PBS/ 0.2% serum/ 0.1% Tween-20). After three rinses using PBS/ 0.2% serum/ 0.1% Tween-20, 100 µl of TMB substrate (TMB reagent set, BD Biosciences) were added and incubated in the dark at room temperature in a wet chamber. Following a one-hour incubation in which the peroxidase catalyzed conversion of the colourless substrate to a blue dye, the reaction was terminated by adding 100 µl of 1 mol/L H₂SO₄ inducing a change to yellow colour. The strength of enzymatic activity and the proportional extinction at a wavelength of 450 nm correlates with the number of anti-EGFR antibodies bound to the EGFR and therefore can be used as measure for the impact of treatments on expression of the EGFR.

**Fig. 6** shows the effects of different doses of cisplatin on EGFR-expression of epithelial cells of a head and neck squamous cell carcinoma (HNSCC) in the *ex-vivo* assay and the cell-based ELISA described in the invention. HNSCC cells were cultured for 72 hours, methanol fixed and stained using the indicated anti-EGFR antibody (Santa Cruz Biotechnology sc-03-G), the biotinylated antibody (BioGenex HK337-5G), and the streptavidin-horseradish peroxidase conjugate (BioGenex CJ-1005-96). TMB was used as substrate and incubated for 30 min before termination by adding an equal volume (100 µl) of 1 mol/l H₂SO₄. The presented data are results of the measurement of optical density of the resulting yellow dye at 450 nm normalized to mean of control. Each measurement at the indicated concentrations of cisplatin or controls was done in five replicates for which mean and standard error of mean are shown.

### Example 6: Demonstration of photoactivation-based disturbance of chemoresponse assays

A MTT assay to demonstrate the photoactivation-based disturbance of chemoresponse assays in regard to effects of docetaxel on proliferation of the epithelial cell line KB (DSMZ ACC 136) was performed.

To address this question, three media, a) the RPMI 1640 without riboflavin and phenol red that is used in the method according to the invention and was manufactured according to our specifications by Biochrom, as well as two different RPMI 1640 charges purchased from Invitrogen which were each containing 532 nmol/L riboflavin, b) a phenol red-free (Gibco 11835), and c) a conventional RPMI 1640 (Gibco 21875) with 13.284 µmol/L phenol red. All media were supplemented with 2 mmol/L L-alanyl-1-glutamine as stabilized glutamine source (Biochrom K0302 Lot 0755 S) and the same amount of 10% (v/v) of heat-inactivated fetal calf serum (FCS) that was purchased from Invitrogen (Lot 41Q7554K) with a yield of 198 nmol/L riboflavin. Hence, the final concentrations of riboflavin in the media were 19.8 nmol/L in the case of the medium according to the method of the invention, and 498.6 nmol/L in the conventional media b) and c) purchased by Gibco. These medium concentrations define the riboflavin concentrations in controls (see below).

In addition to the influences exerted by the different types of media alone, the riboflavin-dependent impact on the outcome of the docetaxel treatment was investigated. For this purpose 100 µl/well of docetaxel dilutions containing threefold the concentration indicated were added into the wells of microwell plates. The following seven docetaxel concentrations were tested: 19.38, 38.75, 77.5, 155, 310, 620, and 1,240 pmol/L.

To examine photoactivation-dependent riboflavin-mediated effects disturbing the outcome of *ex-vivo* chemoresponse-evaluation by conventional cell culture medium, and in particular in the absence of flavin-protecting experimental conditions, varying amounts of riboflavin were added. A stock solution of 200 µM (-)-riboflavin (*CAS 83-68-5,* Sigma R9504, FW=376.37 g/mol, purity >99%, cell culture tested) in phosphate buffered saline was diluted to adjust in each case the highest final concentration in culture (this means: after completion of the assay by addition of the other two components to the final assay volume of 300 µL) to exactly 10 µmol/L. Serial log10-dilutions were made from this concentration in each corresponding medium. These gave final concentrations in cell culture of 10.000, 1.018, 0.12, and 0.03 µmol/L in the case of medium a) (the medium according to the method of the invention), and concentrations of 10.000, 1.449, 0.594, and 0.508 µmol/L using media b) and c).

In general, each combination of riboflavin and docetaxel were tested in duplicate, and controls without additional riboflavin in quadruplicate for each experimental setting in the same 96-well plate. The experimental conditions were as follows: For each of the used media, two different plates were prepared simultaneously and either handled completely under flavin-protecting conditions (illumination only by sodium discharge lamps with an emission wavelength of 589 nm [i]), or in the same bench (Thermo MSC 12) with the installed light inside their working chamber switched on [2]. The dilution of docetaxel and those of riboflavin in the particular medium were prepared for all four plates of each medium type prior to the other working steps under flavin-protecting conditions. Thus the administrations of these dilutions took about 20 min either under flavin-protecting conditions or illuminated conditions. After completed preparation of the plates that thereafter contained 200 µL of both diluted compounds, the plates were incubated 2 h in the incubator to allow for adjustment of the appropriate pH.

In between the stock culture of KB was harvested by trypsination of a 3-days stock culture (exponential growth phase). Stock cultures of KB cells always were grown without any antibiotic or antimycotic in phenol red- and riboflavin-free RPMI 1640 supplemented with 10% (v/v) FCS and were adapted to this medium for more than five passages. They were always handled at room temperature or warmer and were never exposed to light other than the light of a sodium discharge lamp with an emission wavelength of 589 nm for at least five passages. To ensure optimal incubation conditions, the CO₂ concentration therein was constantly kept at 36.5°C, 3.5% CO₂, and a relative humidity at 95%.

After stopping further trypsin digestion by adding a solution of 10% (v/v) of FCS in phosphate buffered saline, an aliquot was taken before partition the KB-cell suspension into three (one third in each tube for washing by centrifugation and afterward re-suspending the cells in their corresponding medium). While washing the cells by centrifugation, the cells where counted using a semi-automated cell counter (Casy, Schärfe Systems). The number of cells then was adjusted to 10⁵/ml in the appropriate volume of completely supplemented RPMI 1640 type a), b) or c). All preparative steps were done under flavin-protecting conditions. 100 µL of this suspension were pipetted into the prepared microtiter plates. Following administration of cell suspension in the first six plates (two plates of each medium type), these plates were transferred into the incubator. In the next step the handling under complete flavin-protecting conditions was finished by switching on the bench's own lighting. Addition of the cell suspensions to the six plates of the second set (two per medium type) was completed within the next 10 min. For these six plates the complete illumination time by the bench's own lighting in sum was just below 30 min.

Following a further incubation of 30 min in the incubator, six plates (one plate out of the identically handled pairs) were put for 30 min on a Styrofoam plate placed inside the lab under flavin-protecting conditions, while the other six plates were put for the same time on a Styrofoam plate inside the neighbouring lab exactly 60 cm behind the closed double-glazed window looking to the north. Following this, the plates were put in the incubator and therein incubated for three days until performing the MTT assay.

The MTT assay is a colorimetric bioassay often used for the measurement of proliferation or even cytotoxicity. This assay rapid and simple assay makes use of the conversion of yellow tetrazolium compounds to their corresponding blue-violet formazan salts by dehydrogenases of viable cells. Consequently, the metabolic capacity of cells within a well to converse MTT to the formazan reflects the number of viable cells. This is measurable via optical density (extinction) at a wavelength of 570 nm (E₅₇₀ nm).

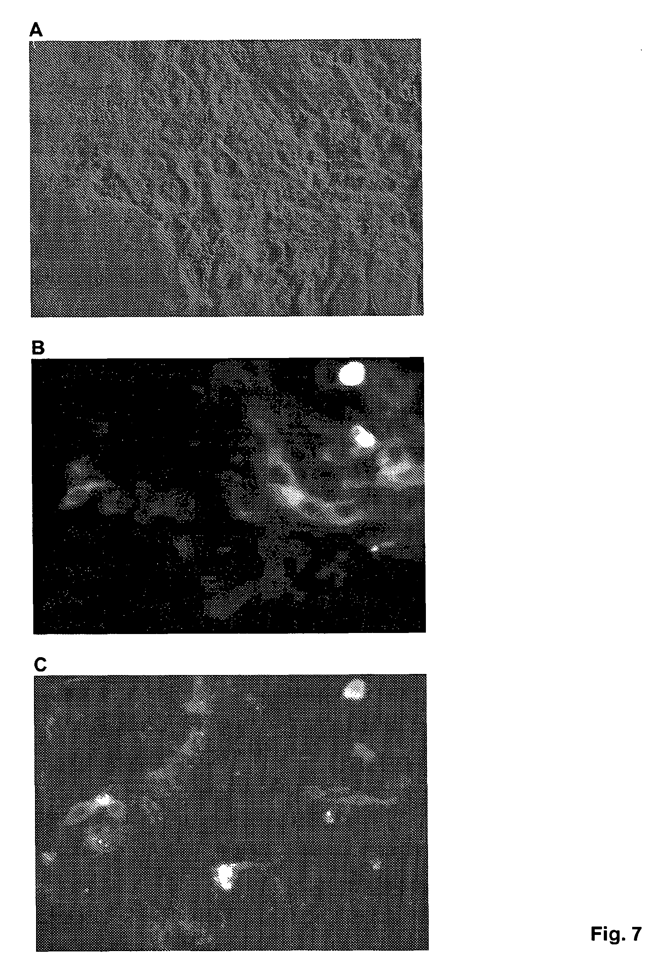

Firstly, the Es_{70 nm}-measurements of controls in plates 01, 05, and 09 (plates that were completely handled under flavin-protecting conditions) showed that the growth of KB cells in the three types of RPMI 1640 were only slightly different (Fig. 7). Moreover, in all measurements with the exception (10 µmol/L riboflavin that were the highest applied concentration) showed comparable E_{570 nm} values in controls. Also the IC₅₀ values for docetaxel were consistently found in the same range of concentrations (circa 200 pmol/L). In case of medium a) (plate 01), IC₅₀ were slightly enhanced if riboflavin concentrations increased up to 1.018 µmol but showed a somewhat different outcome at 10 µmol/L. This resulted in decreased proliferation or even vitality of KB cells and increased toxicity of docetaxel. This led to a different outcome dependent on the reference point chosen, either the individual control (increased IC₅₀ of 256.7 pmol/l which is 128% of the IC₅₀ detected in a) at 20 nmol/L riboflavin) or 224.2 pmol/L (107%) if relating to medium control. (Likewise only slight variability was observed if medium b) was used.) Despite these effects appear to be very marginal if only phenol red-free RPMI 1640 was used, they show in tendency the effects that much clearer can be observed if medium c) is used. But also in this latter case reliable identification of IC₅₀ values for docetaxel appear to be possible if only the medium's riboflavin content is below 10 µmol/L (table 3).

If the preparation of the assays is performed using illumination of the bench's on lighting and even if the illumination time is below 30 min, the outcome of the chemoresponse assay is essentially disturbed. The result is a reduced proliferation of KB cells as observed using all three types of RPMI 1640 (Table 3, Fig. 7). The unacceptable high deviation from the normal outcome is further increased if higher riboflavin concentrations are present. This is made obviously clear if looking at plates 03 or 07. Plate 03 demonstrates that even slight increases in riboflavin concentrations (30 instead 20 nmol/L) are able to disturb IC₅₀ determination in particular if this coincides with exposure to light of shorter wavelengths. Moreover, the dramatically escalated toxicity of 10 µmol/L riboflavin is demonstrated by complete die off of the KB cells making IC₅₀ estimation impossible. Even though the increasing riboflavin content lowered the proliferation, if cells in medium b) and c) during handling were exposed just below 30 min to light of neon lamps containing shorter wavelength light, an complete elapse at the highest riboflavin concentration (10 µmol/L) was not observed. Contrary, concentrations above 594 nmol/L riboflavin in general led to complete dying of all KB cells whenever they were exposed to daylight (plates 02, 04, 06, 08, 10, and 12) even if this was for 30 min in the afternoon of a cloudy day in early spring (30^{th} of March).

Taken together, only the procedure according to the method of the invention fits best the requirements of reliable chemoresponse testing. This is based on the complete avoidance of short wavelength light independent of its source or comparably low exposure times even in the long run. As demonstrated by the representative data for KB cells, the homogeneity in IC₅₀ determination and other parameters allowing for convenient description of dose-response effects are further improved by a reduction of the content of riboflavin in cell cultures to only the concentration that is essential for physiological normal cell function and sufficient to guaranty maintenance of proliferation.

Table 3 and table 4 show the outcome of these investigations.

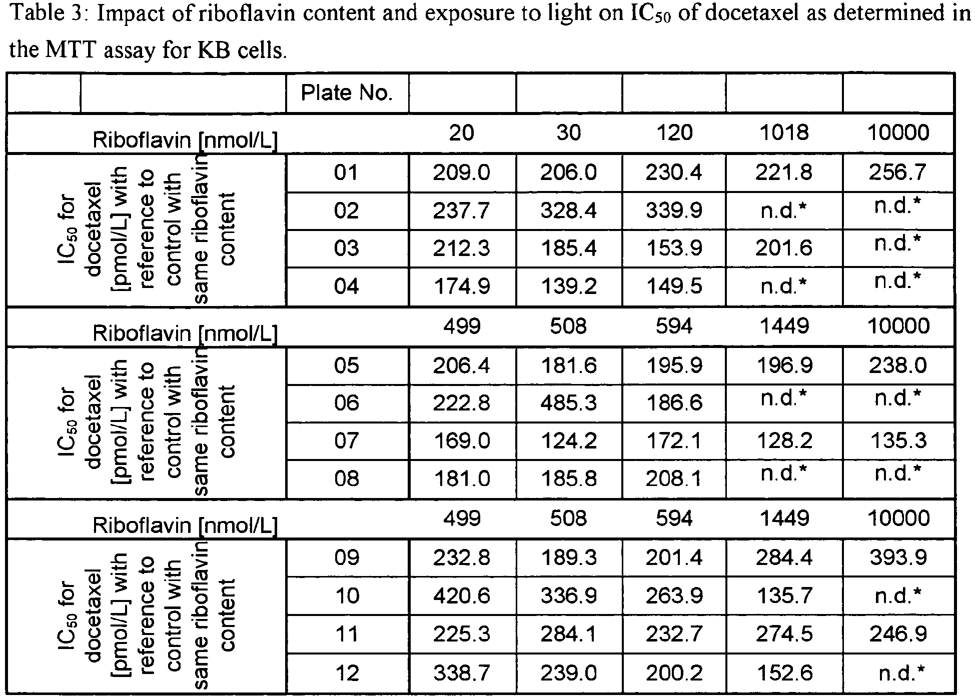

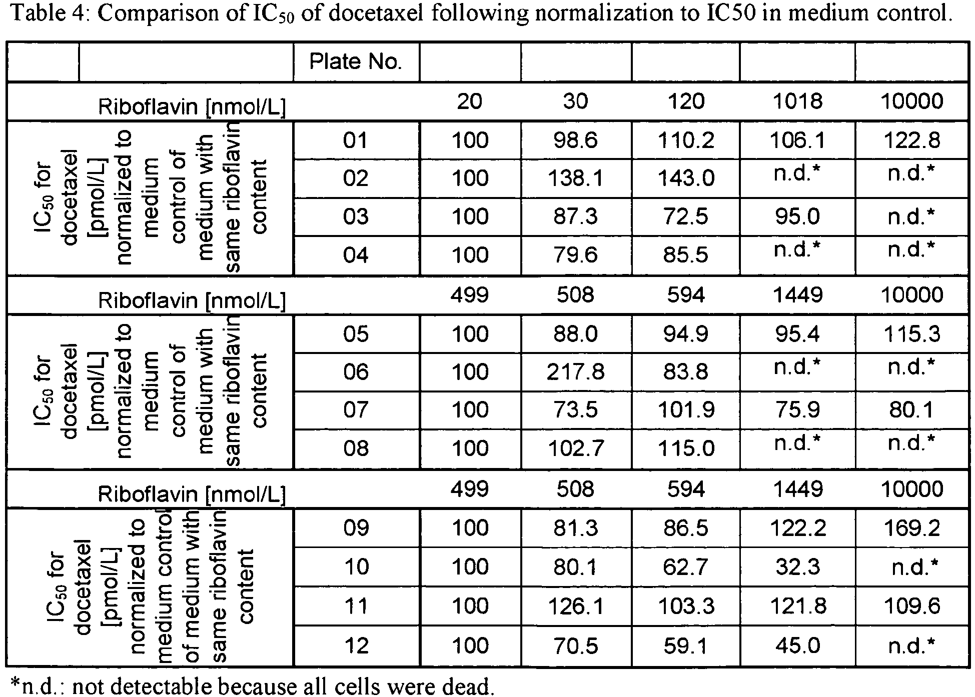

### Example 7:

After obtaining patient's informed consent, a biopsy was taken from histologically confirmed primary head and neck squamous cell carcinoma during definitive surgical treatment under general anesthesia and, after storing for less than an hour in test tubes containing antimycotics and antibiotics (nystatin, penicillin, streptomycin, and amikacin), were cleaned and weighed and processed immediately. Thereafter the sample of 384 mg was mechanically disintegrated by mincing to tissue pats of about 0.1 to 3 mm³ and immersed in 30 ml of pre-warmed riboflavin-free RPMI 1640 medium without phenol red and containing 14.5 mmol/l HEPES, 13.5 mmol/l NaHCO₃, stabilized glutamine, 10% (v/v) fetal calf serum, the antibiotics penicillin, streptomycin and amikacin, as well as the antimycotic nystatin. The tumor cells were always handled at room temperature or warmer and never exposed to light other than the light of a sodium discharge lamp with an emission wavelength of 589 nm. Immediately after mechanical disintegration 120 U/ml collagenase type IV of *Clostridium histolyticum* (EC 3.4.24.3, type IV) were added and the mixture incubated for 16 h at 36.5°C in brown glass bottles in an incubator. To ensure optimal incubation conditions, the CO₂ concentration therein was constantly kept at 3.5% and relative humidity at 95%. Following enzymatic disintegration by collagenase the digest was centrifuged for 10 min at 1200 rpm, the resulting pellet was carefully re-suspended in fresh culture medium supplemented as described but without collagenase. In this setting, the chemoresponse assay was performed in a 48-well microtiter plate coated with 5 µg/ml of fibronectin (Sigma F1141) in phosphate buffered saline at pH of 7.35. Thereafter, a defined amount of digested tumor (3.8 mg in 300 µl tumor medium) was added to microwells 300 µl of culture medium containing twice the concentration of cisplatin or no cytostatic substance at all (control). *Ex-vivo* testing was performed 72 h using the same culture conditions as during the collagenase digest.

After 72 hours of incubation, the wells were rinsed twice with phosphate buffered saline, pH 7.8, and the adherent cells and cell colonies were fixed with ice-cold 90% ethanol for 10 min.

Fixed specimens were exposed for 5 min to PBS/ 0.1% Tween-20^{®} (Polysorbate 20) before preincubation with 1% serum in PBS/ 0.1% Tween-20 for 45 min, followed by overnight incubation at 4°C with the primary antibody (anti-cytokeratin; SantaCruz sc-8018, C11) diluted 1:400 in PBS/ 0.1% Tween-20 supplemented with 0.2 % serum. After three rinses for 10 min each with PBS/ 0.2 % Serum/ 0.1% Tween-20, specimens were incubated for another 2 h at room temperature in the dark with the secondary antibody (anti-mouse IgG coupled with Cy2 fluorescence dye, Jackson ImmunoResearch, Cambs, UK) in a 1:400 dilution in PBS/ 0.2 % Serum/ 0.1 % Tween-20 followed by three rinses with PBS/ 0.2 % Serum/ 0.1 % Tween-20. Thereafter and with the intention to avoid unspecific staining, a 1:400 dilution of mouse serum was added to block free paratops (mouse IgG binding structures in the antibody) of the Cy2-labeled secondary antibody that was used in the step before for cytokeratin staining.

After 2 h incubation at room temperature, the primary labeled anti-vimentin-Cy3 antibody (Sigma C9080) was added in 200 µl of a 1:400 dilution in PBS/ 0.2 % Serum/ 0.1 % Tween-20and incubated additional 2 h. Following three rinses with PBS/ 0.1 % Tween-20, and a final rinse with distilled water for 2 min, the test plate was air dried. The fluorescence signals were imaged using the Zeiss Axiovert 200M inverted fluorescence microscope (Zeiss, Jena, Germany) equipped with Zeiss filter sets 44 and 43HE for detection of Cy2 and Cy3, respectively.

The evaluation of the colonies is possible using every given fluorescence microscope but is eased by the use of dedicated filter sets. Colonies of epithelial origin can be clearly distinguished from stromal cells without fluorescent staining (compare Fig. 7A and 7B). Moreover, the use of vimentin allows for staining of motile cells, which might be derived from epithelial cells but are undergoing epithelial to mesenchymal transformation. The test is utilized by separately counting of colonies of different types (stained epithelial, unstained stromal cells), the numbers of colonies in the different treatments (*e.g*. doses of chemotherapeutics) are recorded and kept for further analysis. The same applies in particular for the number of cells double stained for cytokeratin and vimentin which represent motile cells and thus might correlate with metastasis.

**Fig. 7** demonstrates the immunodetection of epithelial cell colonies formed *ex vivo* according to the invention by immunofluorescence staining following exposure to the cytostatic drug cisplatin at a concentration of 6.667 µmol/L. Shown is a parallel presentation of phase contrast (images A) and using the particular fluorescence filters for either Cy2 fluorescence (image B) or Cy3 (image C). B: Identification of epithelial cells in the particular fluorescence image by anti-cytokeratin staining. C: Detection of vimentin expressing cells by direct staining using a Cy3-labeled anti-vimentin antibody. Notice the double-positive cell that can be found just in the middle in the left side of the image. Magnification: 400-fold.

## Claims

1. Method for the *ex-vivo* evaluation of the response of a tumor to conditions to be tested, in particular a tumor treatment regime, in an sample of isolated tumor, wherein the tumor cells are from epithelial origin, comprising:
a.) Placing a freshly isolated tumor sample in a container, which contains antibiotics and preferably antimycotics keeping the sample at a temperature above 10 °C, preferably above 15 °C,
b.) Breaking up, preferably mechanically disintegrating, the tumor sample,
c.) Placing the pieces of the tumor sample into cell culture medium that contains collagenase, within a maximum of 12 hours, preferably 5 hours, upon isolation of the tumor, incubating for at least 10 hours, followed by washing and re-suspending the cells and tissue pieces in cell culture medium,
d.) Plating the cells and tissue pieces into wells that are coated with extra cellular matrix components, incubating under the conditions of the tumor treatment regime or in presence of a substance to be tested,
e.) Determining the number of cells and/or the number of colonies and/or the sum response of cells of epithelial origin by performing a cytokeratin staining,
f.) Using a cell culture medium in the steps c.) to e.) that contains less than 100 nmol/l flavin and that does not contain phenol red,
g.) Performing the steps c.) to e.) in the absence of light of a wavelength below 520 nm.

2. Method according to claim 1, **characterized in that** the tumor sample of epithelial origin is taken from a carcinoma, preferably chosen from squamous cell carcinomas, papillomas, adenomas or adenocarcinomas, anaplastic carcinomas (*e.g*. of head and neck cancer), adnexal and skin appendage cancer, cystic, mucinous, and serous carcinoma, ductal, lobular and medullary cancer, acinar cell cancer, complex epithelial cancer, gonadal cancer, paragangliomas and glomus tumors, nevi and melanomas.

3. Method according to claim 1 or 2, **characterized in that** the tumor treatment regime is chosen from chemotherapy, immunotherapy and radiation therapy or other physical therapies as well as a combination of those methods.

4. Method according to one of the claims 1 to 3 wherein the isolated tumor sample has a weight of at least 50 mg.

5. Method according to one of the claims 1 to 4, wherein a control cell line or several control cell lines are subjected in parallel to the tumor treatment regime.

6. Method according to claim 5 used for purposes of quality assurance, quality control, control of reproducibility, standardization and/or maintenance of testing conditions.

7. Method according to one of the claims 1 to 6, wherein the number of cells and/or the number of colonies and/or the sum response of cells of endothelial origin or originate from other stromal cell types (like fibroblasts) are determined in parallel.

8. Method according to one of the claims 1 to 7, wherein the number of cells and/or the number of colonies of carcinoma stem cells are determined in parallel, preferably staining with one or several antibodies, that react specific with human stem cell markers and/or carcinoma stem cells and/or by negative staining with DNA dyes.

9. Method according to one of the claims 1 to 8, wherein the cell-type specific expression of protein is analyzed using immunofluorescent staining using labeled primary or secondary antibodies or immunoreagents.

10. Method according to one of the claims 1 to 9, wherein the expression of protein is analyzed using cell-based enzyme-linked immunosorbent assay (ELISA) using enzyme-labeled primary or protein-specific primary and enzyme-labeled secondary antibodies or immunoreagents.

11. Use of a Kit comprising:
a.) cell culture medium containing less than 100 nmol/l, preferably less than 50 nmol/l, flavin and not containing phenol red,
b.) an anti-cytokeratin antibody and preferably a labeled secondary antibody for the *ex vivo* evaluation of a tumor treatment regime in an isolated tumor sample in a method according to one of the claims 1 to 10.

12. Use according to claim 11, whereas the kit contains additionally at least one of the following additional components:
c.) an antibody, that reacts specific with human endothelial cells and preferably a labeled secondary antibody to detect the anti-endothelial cell antibody,
d.) an antibody, that reacts specific with human fibroblast cells and preferably a labeled secondary antibody to detect the anti-fibroblast antibody
e.) a DNA dye and/or one or several antibodies, that react specific with human stem cell markers and/or carcinoma stem cells.

13. Use according to one of the claims 11 to 12, whereas the kit contains additionally a control cell line from epithelial origin and if applicable a control cell line derived from endothelial origin and/or fibroblast cells or a fibroblast cell line and/or a cell line with stem cell properties.

14. Use of the method according to one of the claims 1 to 10 in medicine, in particular for the pre-therapeutic evaluation of the responsiveness of a tumor to a tumor treatment regime, and in pharmacy, in particular for the preclinical evaluation of a tumor treatment regime.

## Patentansprüche

1. Verfahren für die ex vivo-Beurteilung der Reaktion eines Tumors gegenüber Testbedingungen, insbesondere eines Tumorbehandlungsregimes, in einer isolierten Tumorprobe, wobei die Tumorzellen epithelialen Ursprungs sind, umfassend:
a.) Platzieren einer frisch isolierten Tumorprobe in einen Behälter, der Antibiotika und bevorzugt Antimykotika enthält, und Aufbewahrung der Probe bei einer Temperatur oberhalb 10 °C, vorzugsweise über 15 °C,
b.) Aufbrechen, vorzugsweise mechanische Zerkleinerung, der Tumorprobe,
c.) Platzieren der Stücke der Tumorprobe in Zellkulturmedium, das Kollagenase enthält, innerhalb von höchstens 12 Stunden, vorzugsweise 5 Stunden, nach der Isolierung des Tumors, Inkubieren für mindestens 10 Stunden, gefolgt von Waschen und Resuspendieren der Zellen und Gewebestücke in Zellkulturmedium,
d.) Ausplattieren der Zellen und Gewebestücke in Vertiefungen, die mit extrazellulären Matrix-Komponenten beschichtet sind, das Inkubieren unter den Bedingungen des Tumorbehandlungsregimes oder in Anwesenheit einer Substanz, die getestet werden soll
e.) Bestimmen der Anzahl der Zellen und/oder der Anzahl der Kolonien und/oder der Summenreaktion von Zellen epithelialen Ursprungs durch Durchführung einer Zytokeratin-Färbung,
f.) Verwendung eines Zellkulturmediums, das weniger als 100 nmol/l Flavin und kein Phenolrot enthält, in den Schritten c.) bis e.),
g.) Durchführen der Schritte c.) bis e.) in der Abwesenheit von Licht mit einer Wellenlänge unter 520 nm.

2. Verfahren nach Anspruch 1, in, dass die Tumorprobe epithelialen Ursprungs aus einem Karzinom, vorzugsweise ausgewählt aus Plattenepithelkarzinomen, Papillomen, Adenomen oder Adenokarzinomen, anaplastischen Karzinomen (z. B. von Kopf-Hals-Krebs), Karzinomen der Adnexe und der Hautanhangsgebilde, zystischen, muzinösen und serösen Karzinomen, duktalen, lobulären und medullären Krebs, komplexem Epithelkrebs, Gonaden-Krebs, Paragangliomen und Glomustumoren, Nävi und Melanomen, stammt.

3. Verfahren nach Anspruch 1 oder 2, **dadurch gekennzeichnet, dass** das Tumorbehandlungsregime ausgewählt ist aus Chemotherapie, Immuntherapie und Strahlentherapie oder anderen physikalischen Therapien sowie eine Kombination dieser Methoden.

4. Verfahren nach einem der Ansprüche 1 bis 3, wobei die isolierte Tumorprobe ein Gewicht von mindestens 50 mg aufweist.

5. Verfahren nach einem der Ansprüche 1 bis 4, wobei eine Kontroll-Zelllinie oder mehrere Kontroll-Zelllinien parallel dem Tumorbehandlungsregime unterworfen werden.

6. Verfahren nach Anspruch 5 verwendet für Zwecke der Qualitätssicherung, Qualitätskontrolle, Kontrolle der Reproduzierbarkeit, Standardisierung und/oder Aufrechterhaltung von Testbedingungen verwendet wird.

7. Verfahren nach einem der Ansprüche 1 bis 6, wobei die Anzahl der Zellen und/oder die Anzahl der Kolonien und/oder die Summe Reaktion von Zellen endothelialen Ursprungs oder die aus anderen stromalen Zelltypen (z. B. Fibroblasten) stammen, parallel bestimmt werden.

8. Verfahren nach einem der Ansprüche 1 bis 7, wobei die Anzahl der Zellen und/oder der Anzahl von Kolonien von karzinomen Stammzellen parallel bestimmt wird, vorzugsweise durch Färbung mit einem oder mehreren Antikörpern, die spezifisch mit humanen Stammzellmarkern und/oder karzinomen Stammzellen reagieren und/oder durch negative Färbung mit DNA-Farbstoffen.

9. Verfahren nach einem der Ansprüche 1 bis 8, wobei die Zelltyp-spezifische Expression von Protein mittels Immunfluoreszenzfärbung unter Verwendung von markierten primären oder sekundären Antikörper oder Immunreagenzien analysiert wird.

10. Verfahren nach einem der Ansprüche 1 bis 9, wobei die Expression des Proteins mit zellbasierten enzyme-linked immunosorbent assay (ELISA) unter Verwendung von Enzym-markierten primären oder Protein-spezifischen primären und Enzym-markierten sekundären Antikörpern oder Immunreagenzien analysiert wird.

11. Verwendung eines Kit umfassend:
a.) Zellkulturmedium, das weniger als 100 nmol/l, vorzugsweise weniger als 50 nmol/l, Flavin und kein Phenolrot enthält,
b.) ein Anti-Zytokeratin-Antikörper und vorzugsweise ein markierter sekundärer Antikörper,
für die ex vivo-Beurteilung der Reaktion eines Tumorbehandlungsregime in einer isolierten Tumor Probe in einem Verfahren nach einem der Ansprüche 1 bis 10.

12. Verwendung nach Anspruch 11, wobei das Kit zusätzlich mindestens eine der folgenden zusätzlichen Komponenten enthält:
c.) einen Antikörper, der spezifisch mit humanen Endothelzellen reagiert und vorzugsweise einen markierten sekundären Antikörpers zum Nachweis des Anti-Endothel-Zellen-Antikörpers,
d.) einen Antikörper, der spezifisch mit humanen Fibroblasten-Zellen reagiert und vorzugsweise einen markierten sekundären Antikörper zum Nachweis des Anti-Fibroblasten-Antikörpers
e.) einen DNA-Farbstoff und/oder einen oder mehrere Antikörper, die spezifisch mit humanen Stammzell-Markern und/oder karzinomen Stammzellen reagieren.

13. Verwendung nach einem der Ansprüche 11 bis 12, wobei das Kit zusätzlich eine Kontroll-Zelllinie epithelialen Ursprungs und gegebenenfalls eine Kontroll-Zelllinie endothelialen Ursprungs und/oder Fibroblasten-Zellen oder Fibroblasten-Zelllinie und/oder einer Zelllinie mit Stammzell-Eigenschaften enthält.

14. Verwendung des Verfahrens nach einem der Ansprüche 1 bis 10 in der Medizin, insbesondere für die prä-therapeutischen Bewertung der Empfindlichkeit eines Tumors gegenüber einem Tumor Behandlungsregime, und in der Pharmazie, insbesondere für die präklinische Bewertung eines Tumors Behandlungsregimes.

## Revendications

1. Procédé d'évaluation *ex-vivo* de la réponse d'une tumeur à des conditions devant être testées, en particulier un régime de traitement de tumeur, dans un échantillon de tumeur isolée, dans lequel les cellules tumorales sont d'origine épithéliale, comprenant :
a.) un positionnement d'un échantillon de tumeur isolée récemment dans un récipient, qui contient des antibiotiques et de préférence des antimycosiques conservant l'échantillon à une température supérieure à 10°C, de préférence supérieure à 15°C,
b.) une décomposition, de préférence une désintégration de manière mécanique, de l'échantillon de tumeur,
c.) un positionnement des morceaux de l'échantillon de tumeur dans un milieu de culture cellulaire qui contient une collagénase, pendant un maximum de 12 heures, de préférence pendant 5 heures, lors de l'isolation de la tumeur, par incubation pendant au moins 10 heures, suivie d'un lavage et d'une remise en suspension des cellules et des morceaux de tissu dans un milieu de culture cellulaire,
d.) un étalement des cellules et des morceaux de tissu dans des puits qui sont revêtus de composants de matrice extracellulaire, avec incubation dans les conditions du régime de traitement de tumeur ou en présence d'une substance devant être testée,
e.) une détermination du nombre de cellules et/ou du nombre de colonies et/ou de la réponse totale de cellules d'origine épithéliale en effectuant une coloration à la cytokératine,
f.) une utilisation d'un milieu de culture cellulaire lors des étapes c.) à e.) qui contient moins de 100 nmol/l de flavine et qui ne contient pas de rouge de phénol,
g.) une réalisation des étapes c.) à e.) en l'absence de lumière d'une longueur d'onde inférieure à 520 nm.

2. Procédé selon la revendication 1, **caractérisé en ce que** l'échantillon de tumeur d'origine épithéliale est prélevé à partir d'un carcinome, de préférence choisi à partir de carcinomes de cellules de l'épithélium malphigien, de papillomes, d'adénomes ou d'adénocarcinomes, de carcinomes anaplastiques (par exemple d'un cancer de la tête et du cou, d'un cancer d'annexes cutanées et annexiel, d'un carcinome cystique mucineux et séreux, d'un cancer canalaire, lobulaire et médullaire, d'un cancer à cellules acineuses, d'un cancer épithéliale complexe, d'un cancer gonadique, de tumeurs de paragangliomes et de glomus, de naevi et de mélanomes.

3. Procédé selon la revendication 1 ou 2, **caractérisé en ce que** le régime de traitement de tumeur est choisi à partir d'une chimiothérapie, d'une immunothérapie et d'une thérapie par rayonnement ou d'autres thérapies physiques de même que d'une combinaison de ces procédés.

4. Procédé selon l'une des revendications 1 à 3, dans lequel l'échantillon de tumeur isolée présente une masse d'au moins 50 mg.

5. Procédé selon l'une des revendications 1 à 4, dans lequel une lignée cellulaire témoin ou plusieurs lignées cellulaires témoins sont soumises en parallèle au régime de traitement de tumeur.

6. Procédé selon la revendication 5, utilisé à des fins d'assurance qualité, de contrôle qualité, de contrôle de la reproductivité, d'une normalisation et/ou d'une maintenance de conditions de test.

7. Procédé selon l'une des revendications 1 à 6, dans lequel le nombre de cellules et/ou le nombre de colonies et/ou la réponse totale de cellules d'origine endothéliale ou provenant d'autres types cellulaires de stroma (du type fibroblastes) sont déterminés en parallèle.

8. Procédé selon l'une des revendications 1 à 7, dans lequel le nombre de cellules et/ou le nombre de colonies de cellules souches de carcinome sont déterminés en parallèle, de préférence par coloration à l'aide d'un ou de plusieurs anticorps, qui réagissent de manière spécifique à des marqueurs de cellules souches humaines et/ou avec des cellules souches de carcinome et/ou par coloration négative à l'aide de colorants d'ADN.

9. Procédé selon l'une des revendications 1 à 8, dans lequel l'expression spécifique à un type de cellule de protéine est analysée en utilisant une coloration d'immunofluorescence en utilisant des anticorps ou immunoréactifs primaires ou secondaires marqués ou des immunoréactifs.

10. Procédé selon l'une des revendications 1 à 9, dans lequel l'expression de protéine est analysée en utilisant un dosage par la méthode ELISA basé sur les cellules en utilisant des anticorps ou des immunoréactifs primaires marqués par une enzyme ou primaires spécifiques à une protéine et secondaires marqués par une enzyme.

11. Utilisation d'un nécessaire comprenant :
a.) un milieu de culture cellulaire contenant moins de 100 nmol/l, de préférence moins de 50 nmol/l, de flavine et ne contenant pas de rouge de phénol,
b.) un anticorps anti-cytokératine et de préférence un anticorps secondaire marqué pour l'évaluation *ex-vivo* d'un régime de traitement de tumeur dans un échantillon de tumeur isolée conformément à un procédé selon l'une des revendications 1 à 10.

12. Utilisation selon la revendication 11, tandis que le nécessaire contient en outre au moins un des composants supplémentaires suivants :
c.) un anticorps, qui réagit de manière spécifique avec des cellules endothéliales humaines et de préférence un anticorps secondaire marqué pour détecter l'anticorps cellulaire anti-endothélial,
d.) un anticorps, qui réagit de manière spécifique avec des cellules fibroblastiques humaines et de préférence un anticorps secondaire marqué pour détecter l'anticorps anti-fibroblaste,
e.) un colorant d'ADN et/ou un ou plusieurs anticorps, qui réagissent de manière spécifique avec des marqueurs de cellules souches humaines et/ou des cellules souches de carcinome.

13. Utilisation selon l'une des revendications 11 à 12, tandis que le nécessaire contient en outre une lignée cellulaire témoin d'origine épithéliale et, si applicable, une lignée cellulaire témoin d'origine endothéliale et/ou de cellules fibroblastiques ou une lignée cellulaire fibroblastique et/ou une lignée cellulaire avec des propriétés de cellules souches.

14. Utilisation du procédé selon l'une des revendications 1 à 10 en médecine, en particulier pour l'évaluation pré-thérapeutique de la réponse d'une tumeur à un régime de traitement de tumeur, et en pharmacie, en particulier pour l'évaluation préclinique d'un régime de traitement de tumeur.
